# EUROPEAN PATENT APPLICATION

(11) **EP 0 314 094 A2**
(43) Date of publication of application: **03.05.1989**
(21) Application number: 88117832.1
(22) Date of filing: 26.10.1988
(51) Int. Cl.: C12N 15/00, C12P 21/02, A61K 37/02

(54) **Recombinant lymphotoxin**

(30) Priority: 28.10.1987 JP 272034/87
(71) Applicant: Eisai Co., Ltd., Tokyo (JP)
(72) Inventor: Wakabayashi, Toshiaki, Tsukuba-shi Ibaraki (JP); Asada, Makoto, Tsukuba-shi Ibaraki (JP); Nagasu, Takeshi, Tsuchiura-shi Ibaraki (JP); Hasegawa, Yoshikazu, Tsukuba-gun Ibaraki (JP); Shikata, Yasushi, Tsukuba-shi Ibaraki (JP); Kuwada, Manabu, Tsukuba-shi Ibaraki (JP)
(74) Representative: Hansen, Bernd, Dr.rer.nat.

(57) **Abstract**

A recombinant lymphotoxin has an antitumor activity and is defined by the below defined amino acid sequence. A15 to A20 may be eliminated. 
10
Pro Ala Ala A1 Leu A2 A3 Asp A4 A5 A6 Gln
20
Asn Ser Leu A7 A8 A9 A10 Asn Thr A11 A12
30
Ala Phe Leu Gln Asp Gly Phe Ser Leu Ser
40
A13 Asn Ser Leu Leu Val Pro Thr Ser Gly
50
Ile Tyr Phe Val Tyr Ser Gln Val Val Phe
60
Ser A14 A15 A16 A17 A18 A19 A20 A21 Thr
70
Ser Ser Pro Leu Tyr Leu Ala His Glu Val
80
Gln Leu Phe Ser Ser Gln Tyr Pro Phe His
90
Val Pro Leu Leu Ser Ser Gln A22 Met Val
100
Tyr Pro Gly Leu Gln Glu Pro Trp Leu His
110
Ser Met Tyr His Gly Ala Ala Phe Gln Leu
120
Thr A23 A24 A25 A26 Leu Ser Thr His Thr
130
Asp Gly Ile Pro His Leu Val Leu A27 A28
140
A29 A30 Val A31 Phe Gly Ala Phe Ala Leu
 wherein A₁-₃₁ may be each any amino acid.

## Description

The present invention relates to a novel recombinant lymphotoxin derivative, a genetic recombination technique for producing it and a pharmaceutical composition containing it. Accordingly, the present invention is useful in the field. of ethical drugs.

### [Prior Art]

Various humoral factors relating to the biophylactic mechanism are present in a Jiving body. These factors are minor physiologically active substances which are mainly generated by the response of leukocyte, lymphocyte, macrophage or the like to stimulus and include interferon (IFN-α,β and _{γ}),- interleukin (IL 1. 2, 3 and 4), lymphotoxin (LT), tumor necrosis factor (TNF), colony-stimulating factor (G, GM-CSF) and so on. Among them, lymphotoxin and TNF have been each identified as a polypeptide which exhibits a direct anticellular activity on a neoplastic cell system and are generated by the stimulus of mitogen from lymphocyte and macrophage, respectively.

It has already been reported that lymphotoxin or TNF can be produced by cloning the gene of lymphotoxin or TNF and culturing Escherichia coli transformed with a vector containing the cloned gene integrated thereinto (see Nature, 312, 721-724, 1984). Further, it has been confirmed experimentally that the lymphotoxin and TNF thus produced by Escherichia coli each have a necrotic effect on implanted cancer in vivo. For example, it has been confirmed that the cancer due to Meth A Sarcoma implanted to a mouse can be remarkably cured by the intravenous or local administration of TNF (see M.A. Palladino et al., J. Immunology, 138, 4023 (1987)).

However, it has been also confirmed that lymphotoxin and TNF do not exhibit any cytotoxin activity on all kinds of cancerous cells specifically, but there are many cancerous cells which are not affected by them at all (see Sugarman, et al., Science, 230, 943 (1985) and Watanabe et al., J. Cancer Res. (Gann), 76, 1115 (1985)). For example, human bladder carcinoma T-24, human malignant melanoma G-361, colon adenocarcinoma LS 174-T, human Larynx carcinoma HEp2 and human fibrosarcoma HT 1080 are hardly damaged by them in vitro.

Meanwhile, it has been also confirmed that lymphotoxin and TNF exhibit not only an antitumor activity but also other various activities, while lipoprotein lipase-inhibiting activity, IL 1-inducing activity, prostaglandin-inducing activity and procoagulant activity have been reported as such activities. When lymphotoxin and TNF are administered clinically, these activities are presumed to appear as adverse reactions. Actually, pyrexia, ague, trepidation, hypotension. GOT GPT elevation, systemic malaise and ALP elevation were observed in clinical phase I studies of TNF (see Cancer and Chemotherapy, 13, 3491, 1986).

In particular, it is seen in Immunobiology 175, 8, 1987 that hypotension is a dose-limiting factor and it is a bar to administration of a large amount of TNF. It is seen in Proc. Nath. Acad. Sci. USA 84, 4273, 1987 that hypotension caused by TNF can be reduced by administration in advance of a cycioxigenase-inhibiting agent much as indometacinum and ketoprofen. These reports show a possibility that hypotensive action would be based on prostagladin E2-breeding-inducing action using TNF.

Lymphotoxin is classified based on the difference in molecular weight, electrical charge and heat resistance into four groups, i.e., a (molecular weight: 70000 to 90000), β (molecular weight: 25000 to 50000), y (molecular weight: 10000 to 20000) and complex (molecular weight > 200000) (see Paul F. Torrence, Biological Response Modifiers, 293, 1985), among which the groups a and β are further classified into subgroups based on the difference in electrical charge. That is "lymphotoxin" is a generic term for various heterogeneous substances.

Aggarwal et al. reported the amino acid structure of lymphotoxin (see J. B. C., 260, 2334 (1985)), while Gray et al. reported the genetic structure of lymphotoxin and thought that the heterogeneity of lymphotoxin might be partially due to the difference in the number of amino acid residues and in the formation of aggregates.

Further, Granger et al. reported that another lymphotoxin which does not cause immune crossing over with the above lymphotoxin was found (see J. Immunology, 137. 1878, 1986) and that this lymphotoxin was different in the spectrum of anticellular activity from the lymphotoxin, the structure of which had been already elucidated. Further, human tumor necrosis factor (TNF) which has been reported as a factor having an antitumor cell activity is thought to be somewhat different from human lymphotoxin in the spectrum of antitumor cell activity, though TNF exhibits a similarity of 28% in amino acid structure to human lymphotoxin and the receptors for the both on the surface of a tumor cell are the same.

On the basis of the above information, the inventors of the present invention have prepared various polypeptides from lymphotoxin and have examined them with the purpose of finding the amino acid sequence of the region exhibiting an antitumor activity and enhancing the antitumor activity, and then reduction of subsidiary ill effects on its clinical use.

### ( Statement of the Invention )

The inventors have found that the antitumor activity of lymphotoxin resides in an amino acid sequence comprising 143 or 137 amino acid residues and that a new polypeptide which exhibits an improved antitumor activity or exhibits a cytotoxic effect even on lymphotoxin-resistant cancerous cells can be obtained by joining an arbitrary amino acid sequence to the above sequence or by replacing the specified amino acids present in the above sequence with other amino acids. The present invention has been accomplished on the basis of these findings.

Namely, the present invention has been made for the purpose of enhancing the antitumor activity of lymphotoxin and enlarging the 'spectrum of the antitumor activity thereof and discloses a novel lymphotoxin derivative having an amino acid sequence comprising 143 or 137 amino acid residues wherein the specified amino acids present in the sequence may be replaced with one of the specific amino acids. Further, the derivative can be efficiently produced by genetic recombination technique. Thus, the gist of the present invention resides in providing a recombinant lymphotoxin derivative containing the above amino acid sequence.

In addition to the above, the recombinant lymphotoxin provides reduction of prostaglandin E2 breeding-inducing power and reduces subsidiary ill effects on its clinical use.

The invention provides a recombinant lymphotoxin containing an amino acid sequence of 143 or 137 amino acids as defined below.

The invention provides a cDNA coding the recombinant lymphotoxin, an expressive plasmid which contains, as an exogenote, a cDNA coding the recombinant lymphotoxin, a host transformed with an expressive plasmid which contains, an exogenote, a cDNA coding the recombinant lymphotoxin and a pharmaceutical composition which comprises a pharmacologically effective amount of the recombinant lymphotoxin and a pharmacologically acceptable carrier.

Now, the present invention will be described in more detail in the items of "Constitution" and "Process".

### Constitution

The recombinant lymphotoxin derivative of the present invention contains an amino acid sequence represented by the following primary structural formula (hereinafter this sequence will be referred to as "the amino acid sequence according to the present invention): 
Pro Ala Ala A1 Leu A2 A3 Asp A4 A5 A6 Gln
10
20
Asn Ser Leu A7 A8 A9 A10 Asn Thr A11

30
A12 Ala Phe Leu Gln Asp Gly Phe Ser Leu
40
Ser A13 Asn Ser Leu Leu Val Pro Thr Ser
50
Gly Ile Tyr Phe Val Tyr Ser Gln Val Val
60
Phe Ser A14 A15 A16 A17 A18 A19 A20 A21
70
Thr Ser Ser Pro Leu Tyr Leu Ala His Glu
80
Val Gln Leu Phe Ser Ser Gln Tyr Pro Phe
90
His Val Pro Leu Leu Ser Ser Gln A22 Met
100
Val Tyr Pro Gly Leu Gln Glu Pro Trp Leu
110
His Ser Met Tyr His Gly Ala Ala Phe Gln
120
Leu Thr A23 A24 A25 A26 Leu Ser Thr His
130
Thr Asp Gly Ile Pro His Leu Val Leu A27
140
A28 A29 A30 Val A31 Phe Gly Ala Phe Ala

Leu
 wherein A₁ to A₃- may be each any amino acid.

Particularly, an excellent antitumor activity is attained, when A₁ is His, Asn or Leu; A₂ is Ile, Met or Gly; A₃ is Leu, Gln, His, Cys, Ile, Pro, Val, Ser or Gly; A₄ is Pro, Arg, Leu, Gln or Val; As is Ser, Phe or Cys; A₆ is Lys, Asn, Gin, Glu or Asp; A₇ is Leu, Ala or Gln; A₈ is Trp, Phe or Leu; A₉ is Arg or Leu; A₁₀ is Ala, Arg or Glu; A₁ is Asp, Asn, Ala or Arg; A_{1 2} is Arg, Asn or Gly; A₁ is Asn, Arg, Asp, Ala or Ser; A_{1 4} is Gly, Arg, Lys, Pro, Ala, Ser or Thr: A_{1 5} is Lys, Gly, Ala, Thr or Asn; A₁₆ is Ala, Arg, Lys, Glu, Gln, Ser or Gly; A₁₇ is Tyr. Lys, Arg, Cys or Gly; A_{1 8} is Ser, Arg, Lys, Phe. Thr or Ala; A, ₉ is Pro, Lys, Ser, Thr or Gin; A₂₀ is Lys, Asn, Gly or Thr; A₂₁ is Ala, Lys, Ser or Glu; A₂₂ is Lys or Asn; A₂₃ is Gin, Lys, Arg or Gly; A₂₄ is Gly, Asp or Ser: A₂₅ is Asp, Glu or Gln; A₂₆ is Gin, Arg, Pro or Ser; A₂₇ is Ser, Thr, Glu, Phe or Trp: A₂₈ is Pro, Thr, Trp or Tyr; A₂₉ is Ser, Thr or Tyr. A₃₀ is Thr, Val or Phe and A₃₁ is Phe or Tyr.

It is more preferred that A₁, is His; A₂ is Ile; A3 is Gly; A₄ is Pro or Val: As is Ser; A₆ is Lys; A₇ is Leu; As is Trp: A₉ is Arg; A₁₀ is Ala; A₁₁ is Asp; A_{1 2} is Arg; A, ₃ is Asn; A_{1 4} is Gly; A₁ is Lys; A₁₆ is Ala; A₁ is Tyr; A₁₈ is Ser; A₁ is Pro; A₂₀ is Lys; A₂₁ is Ala: A₂₂ is Lys; A₂₃ is Gln; A₂₄ is Gly; A₂₅ is Asp; A₂₆ is Gin; A₂₇ is Ser; A₂₈ is Pro; A₂₉ is Ser; A₃₀ is Thr and A₃₁ is Phe.

When A₁ is His, A₂ is Gly, A₃ is Lys, A₄ is Leu, As is Ala, A₆ is Asp, A₇ is Lys, As is Lys, A₉ is Lys, A₁₀ is Gln. A₁₁ is Asp, A₁₂ is Gln, A_{1 3} is Ser, A_{1 4} is Pro, A. ₅ is Thr, A₁₆ is Phe and A₁₇ is Phe in the above formula, the resulting lymphotoxin derivative contains the same amino acid sequence as the one contained in a so-called lymphotoxin precursor and exhibits the same antitumor activity as the one of lymphotoxin.

The amino acid sequence joined to the amino and carboxyl terminals of the amino acid sequence according to the present invention are not particularly limited, but may be each any one. Examples of the amino acid to be joined to the amino terminal are as follows:
① Met
② Met Lys
③ Met Thr Met Ile Thr Asn Ser Asp Leu Met Arg Arg Arg Lys
④ Met·Thr Met Ile Thr Asn Ser Pro Ala Gin Thr Ala Arg Gln His Pro Lys Met His Leu Ala His Ser Thr Leu Lys
⑤ Met Leu Pro Gly Val Gly Leu Thr Pro Ser Ala Ala Gin Thr Ala Arg Gin His Pro Lys Met His Leu Ala His Ser Thr Leu Lys
⑥ Met Arg
⑦ Met Thr
⑧ Met Ser
⑨ Met Pro
⑩ Met Gin
⑪ Met Ser Thr Leu Lys
⑫no
⑬ Ser Thr Leu Lys

The final object substance of the present invention can be produced by genetic recombination technique. Therefore, both a cDNA coding the recombinant lymphotoxin derivative of the present invention and an expressive plasmid which contains said cDNA as an exogenote in a state joined in such a way as to permit the control and expression thereof in a selected host are intermediates necessary for the production of the final object substance of the present invention. Therefore, they can unite with the final object substance to constitute the present invention, because all of them aim at overcoming the common problem. Particular examples of the expressive plasmid include the following discriminatively represented members:

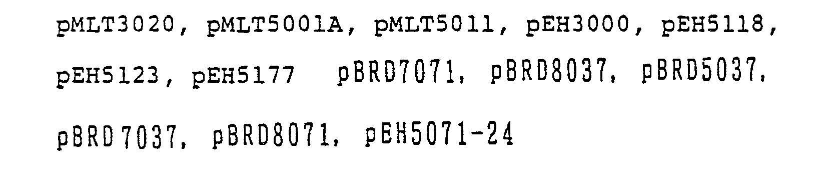

all of which will be described in Examples hereinbelow. Further, a host transferred with said expressive plasmid can be united similarly to the above cDNA and expressive plasmid to constitute the present invention. The host to be transformed includes Escherichia coli and animal cells, while BHK cell is particularly preferably used. Examples of the transferred Escherichia coli include the following discriminatively represented members:

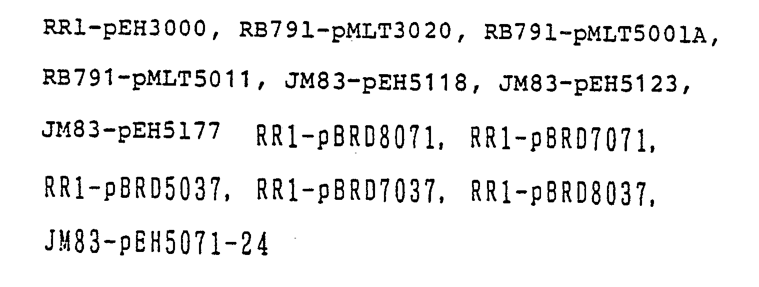

all of which will be described in Examples and have been deposited with Fermentation Research Institute. Their deposition numbers are FERM P-9558, 9559, 9560, 9561, 9562. 9563, 9564 on September 4, 1987. 10254 on August 29. 1988. 10283 on September 16, 1988, 10251 on August 29, 1988, 10284 on September 16. 1988. 10252 on August 29 1988 and 10160 on July 29, 1988, respectively.

The invention provides another recombinant lymphotoxin containing an amino acid sequence of 137 amino acids defined below. This is one of the above defined recombinant lymphotoxin from which A15 to A20 have been eliminated. That is, a recombinant lymphotoxin derivative containing, in its molecule, an amino acid sequence having the following primary structural formula: 
Pro Ala Ala A1 Leu A2 A3 Asp A4 A5 A6 Gln
Asn Ser Leu A7 A8 A9 A10 Asn Thr A11 A12
Ala Phe Leu Gln Asp Gly Phe Ser Leu Ser
A13 Asn Ser Leu Leu Val Pro Thr Ser Gly
Ile Tyr Phe Val Tyr Ser Gln Val Val Phe
Ser A14 A21 Thr Ser Ser Pro Leu Tyr Leu
Ala His Glu Val Gln Leu Phe Ser Ser Gln
Tyr Pro Phe His Val Pro Leu Leu Ser Ser
Gln A22 Met Val Tyr Pro Gly Leu Gln Glu
Pro Trp Leu His Ser Met Tyr His Gly Ala
Ala Phe Gln Leu Thr A23 A24 A25 A26 Leu
Ser Thr His Thr Asp Gly Ile Pro His Leu
Val Leu A27 A28 A29 A30 Val A31 Phe Gly
Ala Phe Ala Leu
 in which A, to A₁₄ and A₂₁ to.A₃₁ each are an amino acid.

It is preferable that A1 is His, Asn or Leu; A2 is Ile, Met or Gly; A3 is Leu, Gin, His, Cys, Ile, Pro, Val, Ser or Gly; A₄ is Pro, Arg, Leu. Gln or Val; As is Ser, Phe or Cys; A₆ is Lys, Asn, Gln, Glu or Asp; A₇ is Leu. Ala or Gln; A₈ is Trp, Phe or Leu: A₉ is Arg or Leu; A₁ is Ala, Arg or Glu; A₁₁ is Asp, Asn, Ala or Arg; A₁₂ is Arg, Asn or Gly; A₁ is Asn. ARg, Asp, Ala or Ser; A, is Gly, Arg, Lys, Pro, Ala, Ser or Thr; A₂₁ is Ala, Lys, Ser or Glu: A₂₂ is Lys or Asn; A₂₃ is Gin, Lys, Arg or Gly; A₂₄ is Gly, Asp or Ser; A₂₅ is Asp, Glu or Gin: A₂₆ is Gln, Arg, Pro or Ser; A₂₇ is Ser, Thr, Glu, Phe or Trp; A₂₈ is Pro, Thr, Trp or Tyr; A₂₉ is Ser, Thr or Tyr. A₃₀ is Thr, Val or Phe and A₃₁ is Phe or Tyr.

It is more preferable that A1 is His; A2 is Ile; A3 is Gly; A₄ is Pro or Val; As is Ser; A₆ is Lys; A₇ is Leu; A₈ is Trp; A₉ is Arg; A₁ is Ala; A₁₁ is Asp; A₁₂ is Arg; A₁₃ is Asn; A_{1 4} is Gly; A₂₁ is Ala; A₂₂ is Lys; A₂₃ is Gln; A24. is Gly; A₂₅ is Asp; A₂₆ is Gin; A₂₇ is Ser; A₂₈ is Pro; A₂₉ is Ser; A₃₀ is Thr and A_{3'} is Phe.

When A1 is His, A2 is Ile, A3 is Gly, A4 is Pro or Val, A5 is Ser, A6 is Lys, A7 is Leu, A8 is Trp, A9 is Arg. A10 is.Ala, A11 is Asp, A12 is Arg, A13 is Asn, A14 is Gly, A21 is Ala, A22 is Lys, A23 is Gin, A24 is Gly, A25 is Asp, A26 is Gin, A27 is Ser, A28 is Pro, A29 is Ser, A30 is Thr and A31 is Phe in the above formula, the resulting lymphotoxin derivative contains the same amino acid sequence as the one contained in a so-called lymphotoxin precursor and exhibits the same antitumor activity as the one of lymphotoxin.

Expressive plasmid containing the recombinant lymphotoxin comprising 137 amino acids includes pEH8084, pBRD8050, pEH5050, pEH6084, pEH7084. Transferred Escherichia coli includes JM83-pEH 6084, JM83-pEH7084. JM83-pEH8084, RR1-pBRD 8050, which are each deposited at Fermentation Research Institute with deposition numbers FERM P-10041, 10040, 10042 on May 27, 1988 and 10253 on August 29, 1988, respectively.

Although the recombinant lymphotoxin derivative which is the final object substance according to the present invention is a mutant of natural lymphotoxin, as will be described in Examples, it exhibits an activity similar to the one of natural lymphotoxin, i.e., antitumor activity equivalent or superior to the one of natural lymphotoxin. Accordingly, not only natural lymphotoxin is useful as an essential active principle for pharmaceutical compositions and its activity is utilized for therapeutic purpose, but also the recombinant lymphotoxin derivative of the present invention is also useful as an essential active principle for therapeutic pharmaceutical composition utilizing the activity. As will be described in Examples, the activity of the recombinant lymphotoxin of the present invention is 4 times or above as much as that of natural lymphotoxin.

The pharmaceutical composition of the present invention is mainly administered as an intravenous injection. Such an injection may be prepared according to an ordinary method for the preparation of an injection containing a minor physiologically active substance. That is, it can be prepared as follows:
The combinant lymphotoxin derivative of the present invention is. alone or together with a suitable filler or a solubilizing agent, dissolved in water to obtain an aqueous solution. This aqueous solution is aseptically filtered, packed and freeze-dried, followed by the attachment of a separately prepared aqueous solution for dissolution. Thus, an injection of a dissolution-before-using type is obtained.

### Process

The processes for the preparation and assay of the substances according to the present invention will be described.

First, lymphokine cDNA library which is necessary for the production of the substances according to the present invention can be prepared by taking out mRNA from a suitable cell, screening the mRNA to select mRNA which exhibits a lymphotoxin activity in an expression system, subjecting the selected mRNA to reverse transcription, preparing a suitable plasmid from the obtained DNA and transforming Escherichia coli with the resulting plasmid (see Nature, 312. 721 to 724, 1984). For example, as will be described, mRNA is fractionated from a Daudi cell and injected into oocyte of Xenopus. The obtained culture supernatant is examined for the lymphotoxin activity to obtain the objective mRNA by screening. The obtained mRNA is subjected to reverse transcription. A plasmid is prepared from the obtained DNA according to an ordinary method and Escherichia coli ₓ 1776 is transformed with the plasmid.

The Daudi cell is one derived from human Burkitt lymphoma and is generally available as ATCC CCL 213 (see Cancer Res. 28, 1300-1310, 1968).

Then, cDNA coding lymphotoxin is selected from the cDNA library by colony hybridization. The colony hybridization may be carried out according to Wallace's method (see Nucleic Acids Res., 9, 879 (1981)) by using, as a probe, a purified synthetic DNA having an amino acid sequence corresponding to the sequence of eight amino acids of the C-terminal of lymphotoxin, i.e., the following sequence:
GTC TTC TTT GGA GCC TTC GCT CTG

A plasmid is prepared from the positive colony, followed by the determination of the base sequence. Thus. a plasmid containing cDNA of lymphotoxin is selected.

A DNA coding the recombinant lymphotoxin derivative of the present invention can be prepared from the plasmid containing cDNA of lymphotoxin by suitably combining known genetic recombination manipulations. According to the present invention. the elimination of an amino acid sequence in a specified position and the replacement of an amino acid in a specified position are exclusively carried out, so that the site-directed mutagenesis of Zoller and Smith is utilized. That is. cDNA of lymphotoxin is incorporated into a phage vector such as M13 to obtain a double-stranded M13 DNA. Escherichia coli is transformed with the double-stranded M13 DNA. The transformed Escherichia coli is cultured to obtain a single-stranded DNA from the resulting culture medium. This single-stranded DNA is annealed by using a specified synthetic oligonucleotide as a primer to induce the mutagesis. After the site-directed mutagenesis has been completed. Escherichia coli is transformed with the obtained reaction mixture and cultured to obtain a plaque. This plaque is hybridized with a radioactively labeled synthetic oligonucleotide. followed by screening. Thus, a double-stranded M13 phage DNA containing a specific cDNA coding the objective substance as an exogenote can be obtained. If necessary, a specified cDNA may be cut from the phage DNA and joined to an expressive vector suitably selected depending upon the host to be used according to an ordinary method.

The expressive vector may be any commercially available one, while examples thereof include pKK 233-2, pUC 12. pUC 18 (Pharmacia), PUG 130 and PUG 131 (plasmids obtained by replacing the polylinker sites of M13 tg130 and 131 with pUC 12 and 13, respectively). Alternatively, as will be described, a special expressive vector may be prepared by joining suitably selected promoter and terminater for use. Namely, in order to express a gene from a mammalian animal in Escherichia coli. yeast or an animal cell, a promoter must be provided on the upstream side of ATG (Met) which is a signal for initiating the synthesis of protein and a terminater must be joined on the downstream side of a protein synthesis-terminating codon (TAA, TGA. TAG). The promoter for Escherichia coli includes lac, trp, tac, trc, sac, and PL and the one for yeast includes a-factor, TPI, suc, GAL, and PGI, while the one for animal cells includes metallothionein, SV 40 early, and LTR. Particularly, it is necessary in order to express such a gene in Escherichia coli efficiently that a promoter, Shine-Dalgarno sequence (SD sequence) and ATG should be accurately joined and the number of bases present between the SD sequence and ATG must be optimumly selected. In view of these points, an expressive vector for containing a trc promoter, SD sequence and ATG joined in this order wherein ATG is present on the downstream side of the SD sequence with eleven intervening bases and Bgl II site is inserted just before the ATG is prepared and used for Escherichia coli in the Examples which will be described.

The genetic recombination manipulations used in the above-mentioned intermediate stages of the process for producing the substances'according to the present invention are almost known to the public, so that they will be briefly described with references.

The description of Maniatis, T, et al., Molecular Cloning, A Laboratory Manual, Cold Spring Harbor Laboratory 1982 is a guide to the cleavage of plasmid with a restriction enzyme and the separation of the DNA fragment obtained by the cleavage by agarose gel electrophoresis or polyacrylamide gel electrophoresis and the recovery and joining thereof.

For example, the description is applied to a case wherein a plasmid containing a cDNA coding natural lymphokine and an M13 phage vector are each cleaved and the obtained fragments are joinded to prepare a double-stranded M13 DNA.

The transformation of Escherichia coli with a plasmid may be carried out according to the method disclosed in DNA Cloning, vol. 1, Chap. 6, ed. by D. M. Glover, IRL press, 1985.

For example, the method is applied to the transformation of Escherichia coli with a double-stranded M13 DNA containing a cDNA coding natural lymphokine incorporated thereinto or an expressive plasmid containing a cDNA coding a mutant lymphokine incorporated thereinto.

The synthesis of oligonucleotide for the mutagenesis and the screening by the phosphoamidite method and the labeling thereof at the 5 terminal may be carried out according to the methods disclosed in S. L. Beaucage et al., Tetrahedron Lett., 22, 1859 (1981) and A. Maxam and W. Gilbert, Methods in Enzymology, Vol. 65, pp. 499 to 560, Academic Press, 1980, respectively.

The base sequence of DNA may be determined by the dideoxy method disclosed in F. Sanger et al., P. N. A. S. 74. 5463 (1977).

For example, the dideoxy method is applied to a case wherein the base sequence of a single-stranded M13 DNA obtained by mutagenesis and screening is determined to confirm whether the objective mutation is induced or not.

A single- or double-stranded M13 phage DNA may be prepared as follows:
A transformed Escherichia coli is cultured according to the process disclosed in J. Messing, Method in Enzymology 101, 20 to 78, 1983, followed by shaking culture and centrifugation. A double-stranded M13 DNA is obtained from the Escherichia coli present in the precipitate by the method disclosed in Maniatis, T, et al., Molecular Cloning, A Laboratory Manual, Cold Spring Harbor Laboratory, 1982. Separately, 260 µl of a 2.5 M NaCl-20% PEG 6000 mixture is added to 1.3 ml of the supernatant and the obtained mixture is centrifuged with an Eppendorf microtube to obtain a pellet. This pellet is suspended in 120 µl of 10 mM Tris hydrochloride buffer (pH 7.9. 0.1 mM EDTA) and extracted with phenol. Ethanol is added to the extract to form a precipitate. This precipitate is washed with 70% ethanol, dried and suspended in 30 µl of 10 mM Tris hydrochloride buffer (pH 7.9. 0.1 mM EDTA) again to obtain a single-stranded M13 DNA.

The site-directed mutagenesis may be carried out according to the method disclosed in Zoller and Smith, Methods in Enzymology, 100 468 to 500, (1983).

Namely, 1 ug of a single-stranded M13 DNA, 2 µl of an annealing buffer (50 mM Tris hydrochloride, pH 8.0, 0.25 mM MgC1₂), 8 µl of B buffer (0.2 M Tris hydrochloride, pH 7.5, 0.1 M MgC1₂, 0.1 M dithiothreitol), 4 µl of a mixture comprising dATP. dGTP. dCTP and dTTP each in an amount of 2.5 mM, 2 µl of 5 mM rATP, 1 µl of distilled water, 5 unit of T₄ DNA ligase and 5 unit of DNA polymerase I Klenow fragment are added to 20p mol of a synthetic oligonucleotide for mutagenesis which has been labeled with a nonradioactive phosphoric acid at the 5 end to make up to a total volume of 20 µl. The mixture is reacted at 14° C for 3 hours.

Then, the transformation of Escherichia coli with a mutant DNA by the use of the above reaction mixture may be carried out according to the process disclosed in Kramer et al., Cell 38, 879 to 887 (1984). Further, according to the process disclosed in Grunstein and Hogness, P.N.A.S. U.S.A. 72 3961 (1975), the transformed Escherichia coli is mixed with Escherichia coli JM 103 to obtain an Escherichia coli mixture. This mixture is spreaded on an agar culture medium to carry out the culture at 37⁰ C overnight. The formed plaque is transferred to a nitrocellulose filter.

The screening of the plaque on the nitrocellulose filter may be carried out as follows:
The nitrocellulose filter is added to a mixture comprising 6 x SSC (1 x SSC comprises 150 mM NaCI and 15 mM of sodium citrate), 10 x Denhardt's (1 x Denhardt's comprises 0.02% of polyvinylpyrrolidone, 0.02% of Ficoll and 0.02% of bovine serum albumin) and 50 µg of supersonically treated salmon sperm DNA. The obtained mixture is treated at 65⁰ C for 3 hours to form a prehybrid. Then, the resulting filter is mixed with a radioactively labeled synthetic oligonucleotide for mutagenesis under the same buffer condition at the one used above. The obtained mixture is allowed to stand at 65 C overnight to form a hybrid. The resulting filter is washed with 6 x SSC. After the removal of water, a sensitized screen is put on the filter and exposed to an X-ray film to select the objective plaque.

The transformation of a host with an expressive plasmid which has been prepared by cutting a cDNA coding a mutant lymphokine from a double-stranded M13 DNA and joining the cDNA to an expressive vector may be carried out by a suitable process selected depending upon the kind of host to be used. For example, the transformation of BHK cell may be carried out by the method disclosed in Loyter et al., P.N.A.S. USA, 79, 422 (1982). That is, BHK⁵⁷⁰ cell strain (ts, tk-) is transformed with the above expressive plasmid and pSV2-dhfr, while the pSV2-dhfr is disclosed in Sabramani et al., Mol. Cell. Biol. 1, 854 to 864 (1981).

The cloning may be carried out by the limiting dilution method as follows:
After several days have elapsed since the transformation, the medium is exchanged with a Dulbecco-modified Eagle medium containing 200 nM of Amethopterin (Dulbecco-modified Eagle medium, 3.5% of glucose, 7.5% of sodium hydrogen carbonate, 5% of fetal calf serum (FCS), 3 mM of tranexamic acid, 2 mM of glutamine, 200 nM of (+)-Amethopterin) every two or three days for one to two weeks to carry out the limiting dilution. Thus, a productive strain is obtained. This productive strain is incubated on 300 ml of a Dulbetco-modified Eagle medium containing 200 nM of Amethopterin placed in a roller bottle and cultured at 37 C. After three or four days have elapsed since the initiation of the culture, the medium exchange with the same amount of a fresh medium is carried out every day for 3 or 4 weeks to collect the culture supernatants.

In the Examples of the present invention, Escherichia coli JW 83, RRI or RB 791 was used and, after the shaking culture, the strain was disintegrated to collect the supernatant fractions.

The collected supernatant fraction may be purified by suitably combining gel filtration, pigment- adsorptive chromatography and high-performance liquid chromatography etc. and fractinated to select an objective fraction based on lymphotoxin activity.

The lymphotoxin activity, i.e.. the antitumor activity of lymphotoxin is determined as follows:
For example, an LP 3 cell which is a substrain of a mouse fibroblast L cell and is highly sensitive to lymphotoxin is used as an indicator cell. A sample and actinomycin D having a final concentration of 1 µg/ml are added to an LP 3 cell to carry out the culture at 37 C under C0₂ current for 12 to 18 hours. In this step, RPMI 1640 containing inactivated fetal serum in an amount of 10% is used as a culture medium. After the completion of the culture, the dead cells are washed out and the remaining living cells are fixed and stained with a 0.5% solution of crystal violet (methanol : water = 1 : 4). The dyestuff is extracted from the stained cell and examined for absorbance to determine the number of the living cells. The titer is shown by the degree of dilution of a sample which attains 50% lysis of the LP 3 cell. The titer must be always corrected by using a lymphotoxin standard titer sample as a cotrol, because it varies depending upon the growth phase of the LP 3 cell exhibited when a sample is added thereto.

The activity concentration unit "1 U. ml" refers to the one attaining 50% lysis after 24 hours when the above assay is carried out by using an LP 3 cell in a logarithmic growth phase subcultured on a serum-free medium (DM-153 medium).

The test on the cytotoxic effect and proliferation inhibiting effect of LT against a human malignant tumor cell strain is made as follows:
Various cell strains are added to a 96-well microtiter plate at a density of 0.5 to 1 x 10³ well and cultured overnight, followed by the addition of a medium containing an LT sample. The resulting system is cultured under wet conditions in the presence of C0₂ for about one week. The number of living cells is determined by the extraction of crystal violet in a similar manner to the one described above.

Alternatively, the assay may be carried out by the simultaneous use of 0.1 to 0.5 µg/ml of actinomycin D. In this case, a cell strain is added in a density of 2 to 5 x 104. well, followed by the addition of an LT sample and actinomycin D. After 12 to 15 hours, the number of living cells is determined.

The specific activity is estimated by enzyme immunoassay (EIA) as follows: The amount of LT contained in a sample is estimated according to the sandwich method by using an antihuman LT antibody from an LT-immunized rabbit or a'monoclonal antihuman LT antibody generated by a hybridoma prepared from a spleen cell of an immunized mouse by the cell fusion. That is, an antihuman LT antibody (monoclonal or polyclonal) is adsorbed to a plastic well. LT is added to the well to be joined thereto. Further, a monoclonal or polyclonal antibody having a recognition site different from that of the primary antibody is added thereto. This secondary antibody has been preliminarily joined to biotin prior to the addition. Therefore, when avidin bonded to horse radish peroxidase is added to the system, the avidin specifically reacts with the biotin of the secondary antibody to fix the peroxidase to the well in an amount proportional to the amount of the secondary antibody bonded to the LT. The resulting system causes quantitative color development by adding a substrate such as o-phenylenediamine. The LT concentration of the sample is estimated from the absorbance of the system. That is, the LT concentration of a sample containing an unknown amount of LT is estimated by comparing the EIA value of the sample with that of a sample, the LT concentration of which has been determined by amino acid analysis. Further, the antitumor activity of the former is determined by the use of an L-P 3 cell as an indicator in a similar manner to the one described above to thereby determine the specific activity thereof.

( Brief statement of drawing )
Fig. 1 shows the base sequence of the amino acid-coding region of lymphotoxin cDNA.
Fig. 2 is the flow diagram of assembly of pMLT3020.
Fig. 3 is the flow diagram of assembly of pUG5001 and pMLT5001A.
Fig. 4 is the flow diagram of assembly of pUG5004.
Fig. 5 is the flow diagram of assembly of pMLT50111.
Fig. 6 is the flow diagram of assembly of pUCLT3011.
Fig. 7 is the flow diagram of assembly of pEH3000.
Fig. 8 is the flow diagram of assembly of pEH5153.
Fig. 9 is the flow diagram of assembly of plasmid.
Fig. 10 is the flow diagram of assembly of plasmid.
Fig. 11 is the flow diagram of assembly of pEH5073.
Fig. 12 is the flow diagram of assembly of pEH5123.
Fig. 13 is the flow diagram of assembly of pEH5118.
Fig. 14 is the flow diagram of assembly of pEH5071.
Fig. 15 is the flow diagram of assembly of pEH5096.
Fig. 16 is the flow diagram of assembly of pBRD5011.
Fig. 17 is the flow diagram of assembly of pEH8071.
Fig.18 shows the flow diagram of assembly of pTRC3000.
Fig.19 shows the flow diagram of assembly of pTT5001.
Fig.20 shows the flow diagram of assembly of pBRD8071.
Fig.21 shows the flow diagram of assembly of pBRD5037.
Fig.22 shows the flow diagram of assembly of pBRD8037.
Fig.23 shows the flow diagram of assembly of pBRD7037.
Fig.24 shows the flow diagram of assembly of pBRD7071.
Fig.25 is a graph which illustrates the evaluated results of the antitumor activity against PC - 10 cells derived from human pulmonary cancer.
Fig.26 shows test results on anticancer activity to WiDr.
Fig.27 shows test results on PGE₂ breeding-inducing power of Rat's periosteum cells.
(Fig. 28 is omitted)
Fig.29 shows the flow diagram of assembly of pEH5123.
Fig.30 shows the flow diagram of assembly of pUG5004.
Fig.31 shows the flow diagram of assembly of pMLT5011.
Fig.32 shows the flow diagram of assembly of pEH6084.
Fig.33 shows the flow diagram of assembly of pEH7084.
Fig.34 shows the flow diagram of assembly of pEH8084.
Fig.35 shows the flow diagram of assembly of pBRD8050.
Fig.36 shows the flow diagram of assembly of pEH5050.
Fig.37, Fig.38 and Fig.39, respectively,show the graphs illustrating the results in Experimental Example 2 and
Fig.40 shows the graphs illustrating the results in Experimental Example 3.

### [Examples]

The present invention will be further described in more detail by referring to the following Examples.

### Example 1

### Preparation of mRNA

Daudi cells (2 × 10⁸) were suspended in 10 ml of a solution containing 4M guanidinium thiocyanate, 0.5% of sodium N-laurolysarcosine, 25 mM sodium citrate (pH 7.0), 0.1 M mercaptoethanol and 0.1% of antiform (sigma). The ontained suspension was homogenized with a Politron. The obtained homogenate was layered on an 5.7 M aqueous solution of cesium chloride containing 0.1 M EDTA. The resulting system was centrifuged with an ultracentrifuge (SRP 28 A rotor: a product of Hitachi) at the rate of 26000 rpm at 20° C for 23 hours. The obtained RNA precipitate was dissolved in 10 ml of 10 mM Tris HCI (pH 7.4), followed by the addition of 2.5 times as much ethanol. The resulting mixture was allowed to stand at -70 C for one hour and centrifuged to form an RNA precipitate. This precipitate was dissolved in 4.14 ml of 10 mM Tris-HCI (pH 7.4), followed by the addition of 2 ml of 4M potassium chloride. The obtained RNA solution was passed through an oligo(T)cellulose column (02 x 15 cm). The column was sufficiently washed with 0.5 M potassium chloride containing 10 mM Tris-HCL (pH 7.4). Then a 10 mM aqueous solution of Tris-HCI (pH 7.4) was passed through the column to elute mRNA.

### Purification of mRNA

500 µg of the mRNA prepared from Daudi cells was layered on 15 ml of a solution containing 50 mM Tris-HCI (pH 7.4), 0.2 M NaCI and 1 mM EDTA and having a sucrose concentration gradient of from 10 to 28%. The resulting system was centrifuged with an ultra-centrifuge (SRP 28 A rotor: a product of Hitachi) at the rate of 26000 rpm at 20 C for 16 hours and fractionated to obtain fractions each of 50 µl. Ethanol was added to each fraction to form a mRNA precipitate. This precipitate of each fraction was dissolved in sterilized water to obtain a solution. The mRNA concentration of the solution was adjusted to 1 µg.µ.l.

### Screening of mRNA

Serum gonadotrophin (200 U) (Peamex: a product of Sankyo) was injected into a female Xenopus of one year or older. After the lapse of one night, the oocyte was taken out and preserved in a modified barth saline medium (MBS). The mRNA of each fraction prepared above was injected into egg in an amount of 50 to 100 ng egg and cultured in MBS containing 1 mM phenyl-methylsulfonyl fluoride (PMSF) at 25° C for 36 hours. After the removal of a supernatant, the oocyte was homogenized and extracted with MBS containing 1 mM PMSF to obtain a mRNA sample of each fraction. Each sample was examined for lymphotoxin activity and screened to obtain a mRNA fraction exhibiting lymphotoxin activity.

### Preparation of cDNA library

5 µg of the mRNA fraction exhibiting lymphotoxin activity contained int he mRNA expression system prepared by the use of Xenopus oocyte was dissolved in 7 µl of 5 mM Tris-HCI (pH 8.3) and incubated at 65° C for 5 minutes. followed by the addition of 1.4 µg of pcDV-1 DNA (Pharmacia). The obtained mixture was reacted under the following conditions: 50 mM Tris-HCI pH 8.3, 8 mM MgC1₂, 30 mM KCI, 0.3 mM DTT. 2 mM dNTP, reverse transcriptease 5U reaction volume 20 µl.

After the reaction had been carried out at 37 C for 60 minutes, the reaction was stopped by the addition of 2 µl of 0.25 M EDTA. The reaction mixture was treated with phenol/chloroform, followed by the addition of 20 µl of 4 M NH₄OAc and 80 µl of EtOH. The obtained mixture was allowed to stand at -70° C and centrifuged to remove the supernatant. The residue was repeatedly subjected to the same treatment as the one described above. The obtained precipitate ws washed with 75% aqueous EtOH. dried and adjusted to a reaction solution of 10 mM sodium cacodylate, 2.5 mM HEPES (pH 7.6), 0.1 mM dCTP, and 0.2 µg/µl poly (A). Finally, the volume of the solution was adjusted to 20 µl by the addition of 10 U of terminal deoxynucleotidyl transferase (TdT). The reaction was carried out at 37° C for 10 minutes and stopped by the addition of 1 µl of 0.25 M EDTA. The reaction mixture was post-treated by an ordinary method.

The obtained DNA precipitate was dissolved in 11 µl of a 10 mM Tris-HCI solution (pH 7.4) containing 8 mM MgCl₂, 6 mM mercaptoethanol and 10 U of Hind III to carry out the reaction at 37° C for 2 hours. The reaction was stopped by the addition of 1 µl of 0.25 M EDTA. The reaction mixture was post-treated by an ordinary method and dissolved in a mixture comprising 10 µl of a solution of 1 mM EDTA in 10 mM Tris-HCI (pH 7.4) and 3 µl of EtOH.

77 ng of pL-1 DNA (Pharmacia) was added to 11 µl of the DNA solution to obtain a mixture. A solution containing 10 mM Tris-HCI (pH 7.4), 1 mM EDTA and 0.1 M NaCL was added to the mixture to form a solution (120 µl). This solution was allowed to stand at 65 C for 5 minutes and at 42 C for 30 minutes. Then. the following components were added to the resulting solution under cooling with ice to make up to a total volume of 1.2 ml:
20 mM Tris-HCI (pH 7.5), 4mM MgCl₂, 10 mM (NH₄)₂SO₄, 0.1 M KCI, 0.1 mMβ-NAD, 50 µg/mℓ bovine serum albumin and 1 u.g of E. coli ligase, The reaction was carried out at 12° C overnight, followed by the addition of 10 µl of 4 mM dNTP, 10 t of 20 mM β-NAD, 1 u.g of E. coli ligase, 17.5 U of E. coli DNA polymerase I and 120 U of RNase H. The obtained mixture was reacted at 12°C for one hour and then at 25° C for another one hour to obtain a reaction solution. This solution was used in the transformation of E. coli ₓ1776.

That is, E. coli ₓ1776 was cultured in a ₓ1776 medium (Maniatis et al., Molecular cloning) at 37° C, until the absorbance thereof reached 0.2. The cultured E. coli ₓ1776 was gathered to prepare a competent cell according to Maniati's method. The above DNA was added to the competent cell to transform the E. coli x1776. Thus, a cDNA library was obtained.

### Screening of cDNA

In order to select a plasmid containing cDNA coding lymphotoxin from the above cDNA library, DNA corresponding to the eight-amino acid sequence of the C-terminal of lymphotoxin was synthesized and the colony hybridization was carried out by using the DNA as a probe.

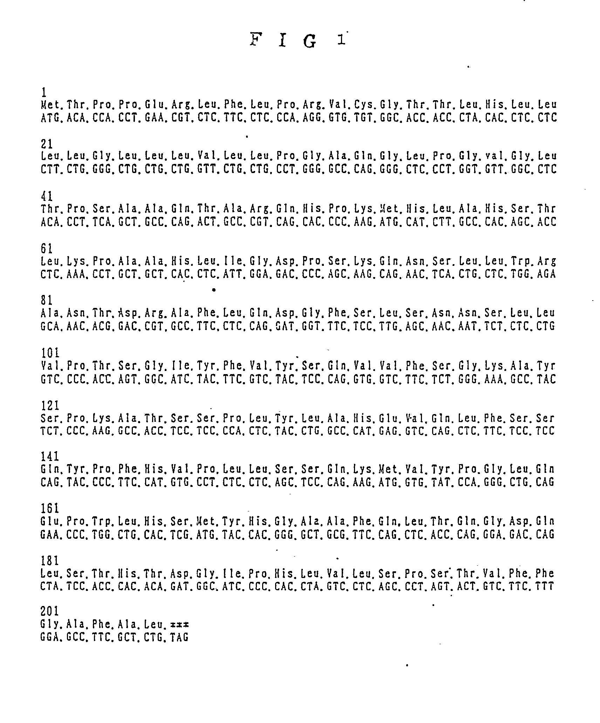

The structure of the synthesized probe is GTC TTC TTT GGA GCC TTC GCT CTG. The probe was synthesized with an automatic solid phase synthesizer (Applied Biosystem) and purified by HPLC. The hybridization was carried out according to Wallace's method (Nucleic Acids Res, 9, 879 (1981)). Among the about 100000 cDNA libraries obtained. 60 libraries were positive colonies. Plasmids were prepared from 3 colonies out of the positive colonies and examined for base sequence. Only one of the plasmids contained lymphotoxin cDNA. The base sequence of the amino acid coding region of the obtained lymphotoxin 'cDNA is shown in Fig. 1.

### Assembly of expressive vector

The plasmid containing the lymphotoxin cDNA prepared above was digested with BamHl to obtain a fragment (about 1.1 kbp) containing the lymphotoxin cDNA. This fragment was inserted into the BamHl site of pUG 131 (a plasmid obtained by replacing the poly linker region of pUC 13 (Pharmacia) with that of M13tgl 31 (Pharmacia)) (Plasmid No. pMLT 2000). The obtained plasmid was digested with Hinc II and Bal I to obtain a fragment (about 1.1 kbp). Separately, pKK233-2 (Pharmacia) was digested with Nco I and treated with Klenow enzyme to obtain a vector. The above fragment (1.1 kbp) was inserted into the vector (Plasmid No. pMLT 2100). The pKK 233-2 contains a trs promoter (one obtained by improving a tac promoter) and can be expressed by the induction of IPTG. Then. the pMLT 2100 was digested with EcoRl and Hind III to obtain a fragment (about 1.5 kbp) containing the lymphotoxin cDNA. This fragment was inserted into M13 mp10 double-stranded DNA (Pharmacia), followed by the transfection thereof into E. coli JM103. According to an ordinary method, single-stranded DNA was prepared to carry out the in vitro metagenesis thereof by using the following synthetic oligonucleotide:

### GCT TGC TGG GAT CCA TGA GAT CTG TTT CCT

The site-directed mutagenesis was fundamentally carried out by Zoller's method (see Method in Enzymology, 100, 468 (1983)). Namely, 1 µg of the template DNA (single-stranded DNA), 2 µl of 5 x annealing buffer(50 mM Tris-HCI, pH 8.0, 25 mM MgCl₂), 8 µl of B buffer (0.2 M Tris-HCI pH 7.5, 0.1 M MgCl₂, 0.1 M DTT), 4 µl of 2.5 mM dNTP, 2 µl of 5 mM ATP, 1 µl of distilled water, 5 U of T4 DNA ligase, 5 U of DNA polymerase I and Klenow fragment were added to 20 p mol of the above synthetic oligonucleotide labeled with nonradioactive phophoric acid at the end to make up to a total volume of 20 µl. The obtained mixture was reacted at 14° C for 3 hours and transformed E. coli JM103 (see Kramer et al., Cell 38, 879 (1984)).

The transformed bacteria were mixed with E. coli JM103. The obtained mixture was spreaded on an agar medium and cultured at 37° C overnight to form a plaque. This plaque was transferred to nitrocellulose (Grunstein et al., PNAS 72, 3961 (1975)) and subjected to the hybridization test with a ³²P-labeled synthetic oligonucleotide to select a phage plaque wherein mutagenesis was induced. A double-stranded DNA was prepared form the phage plaque thus selected and digested with EcoRl and Hind III to obtain a DNA fragment. This fragment was inserted into a plasmid prepared by the digestion of pKK233-2 with EcoRl and Hind III (Plasmid No. pMLT 3020):
The pMLT 3020 is an expressive vector which initiates the synthesis of protein from the ATG on the downstream side of the Shine-Dargaro sequence of the trc promoter with eleven intervening nucleotides, which is digested with Bgl II to permit the insertion of an arbitrary gene into a position on the downstream side of the promoter and which is digested with BamHl to permit a gene into a position on the downstream side of the initiator codon ATG. The pMLT 3020 can code the polypeptide corresponding to the one obtained by removing the seven amino acid residues from the NH₂-terminal of the polypeptide of the present invention. The DNA sequence around the NH₂-terminal is as follows:
Trc promoter region

GTTGACAATTAATCAACCGGCTCGTATAATGTGTGGAATTG

SD Bgl II
TGAGCGGATAACAATTTCACACAGGAAACAGATCTC
Met Asp8 Pro9 Ser10 ...
ATG GAT CCC AGC ...
BamHI

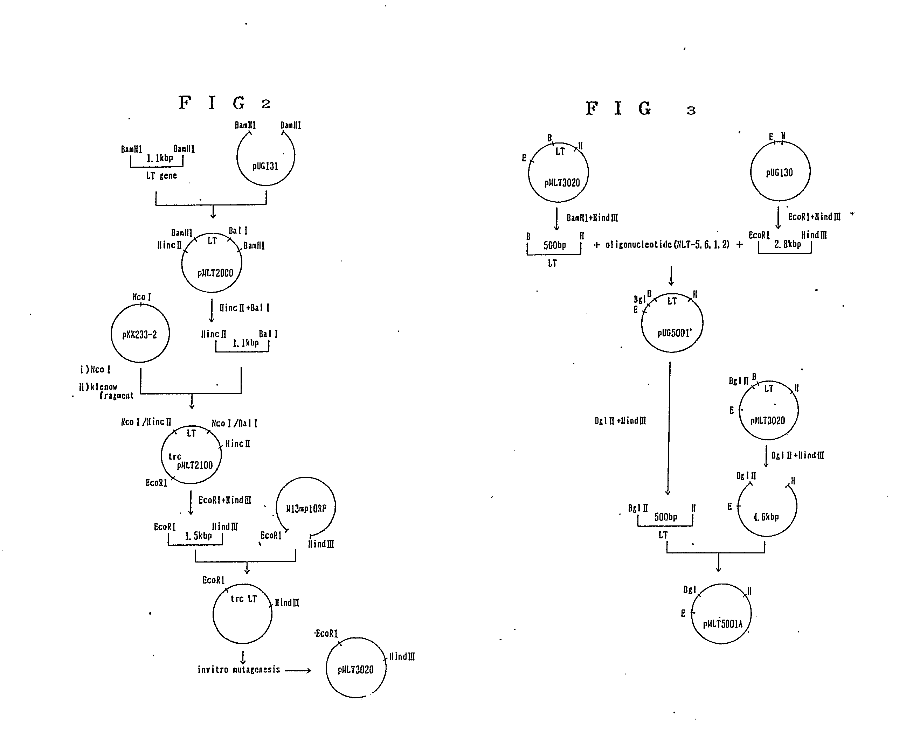

The flow diagram of the assembly of pMLT 3020 is shown in Fig. 2.

### Example 2

pMLT3020 was digested with BamHl and Hind III to recover a DNA fragment of about 500 bp by agarose gel of electrophoresis. Separately, the oligonucleotide which will be described was synthesized and phosphorylated at the 5' terminal with polynucleotide kinase (NEB) and ATP. The phosphorylated oligonucleotide, the above DNA fragment of 500 bp and a vector obtained by the digestion of pUG130 with EcoRl and Hind III were enzymatically joined (Plasmid No. pUG5001)

5' AATTCAGATCTCATGAAA 3' NLT-5
3' GTCTAGAGTACTTTGGGCGC 5' NLT-6
5' CCCGCGGCTCATCTGATCGGG 3' NLT-1
3' CGAGTAGACTAGCCCCTAG 5' NLT-2

After the termination of the reaction, E. coli JM83 was transformed according to an ordinary process to select a white colony on an X-Gal-containing agar plate. Then, the plasmid pUG5001 was digested with Bgl II and Hind III to recover a DNA fragment of about 500 bp by agarose gel electrophoresis. This fragment was enzymatically joined to DNA (about 4 kbp) prepared by the digestion of pMLT3020 with Bgl II and Hind III (Plasmid No. pMLT5001A). The flow diagram of this assembly is shown in Fig. 3.

### Example 3

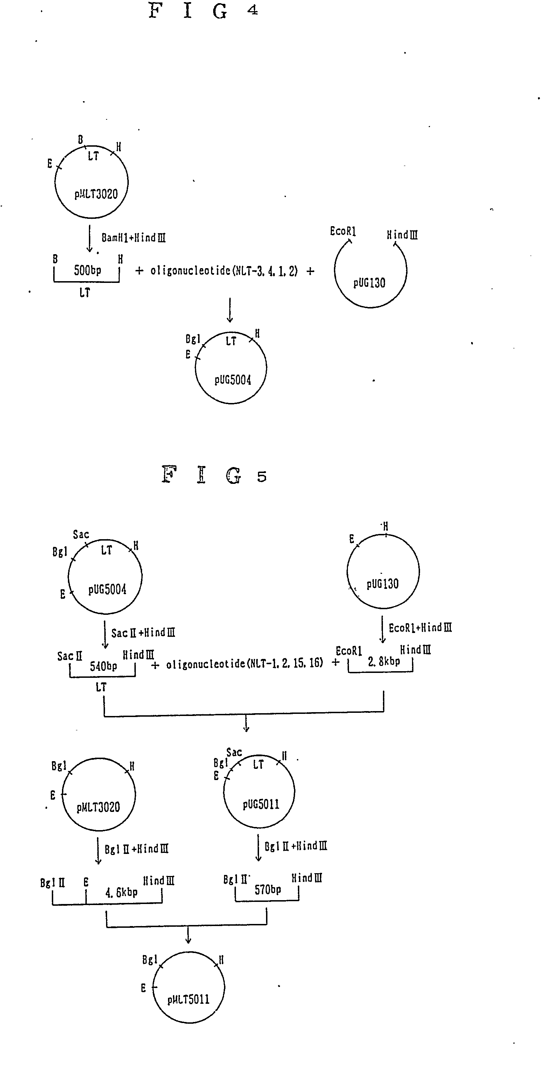

A DNA fragment of about 500 bp prepared by the digestion of pMLT3020 with BamHl and Hind III, a synthetic oligonucleotide phosphorylated at the 5 terminal which will be described and a vector prepared by the digestion of pUG130 with EcoRl and Hind III were enzymatically joined (Plasmid No. pUG5004). The flow diagram of this assembly is shown in Fig. 4.

5' AATTCAGATCTCATGCGTCGTCGTAAA 3' NLT-5
3' GTCTAGAGTACGCAGCAGCATTTGGGCGC 5' NLT-4
5' CCCGCGGCTCATCTGATCGGG 3' NLT-1
3' CGAGTAGACTAGCCCCTAG 5' NLT-2

### Example 4

Plasmid pUG5004 was digested with Sac II and Hind III to obtain a DNA fragment of about 540 bp. This fragment, the synthetic oligonucleotide phosphorylated at the 5 terminal which will be described and another DNA fragment of about 2.8 kbp prepared by the digestion of plasmid pUG 130 with EcoRl and Hind III were enzymatically joined (Plasmid No. pUG5011).

Then, the plasmid pUG5011 was digested with Bgl II and Hind III to obtain a DNA fragment of about 570 bp. This fragment and another DNA fragment of about 4.6 kbp prepared by the digestion of plasmid pMLT3020 with Bgl II and Hind III were enzymatically joined (Plasmid No. pMLT5011).

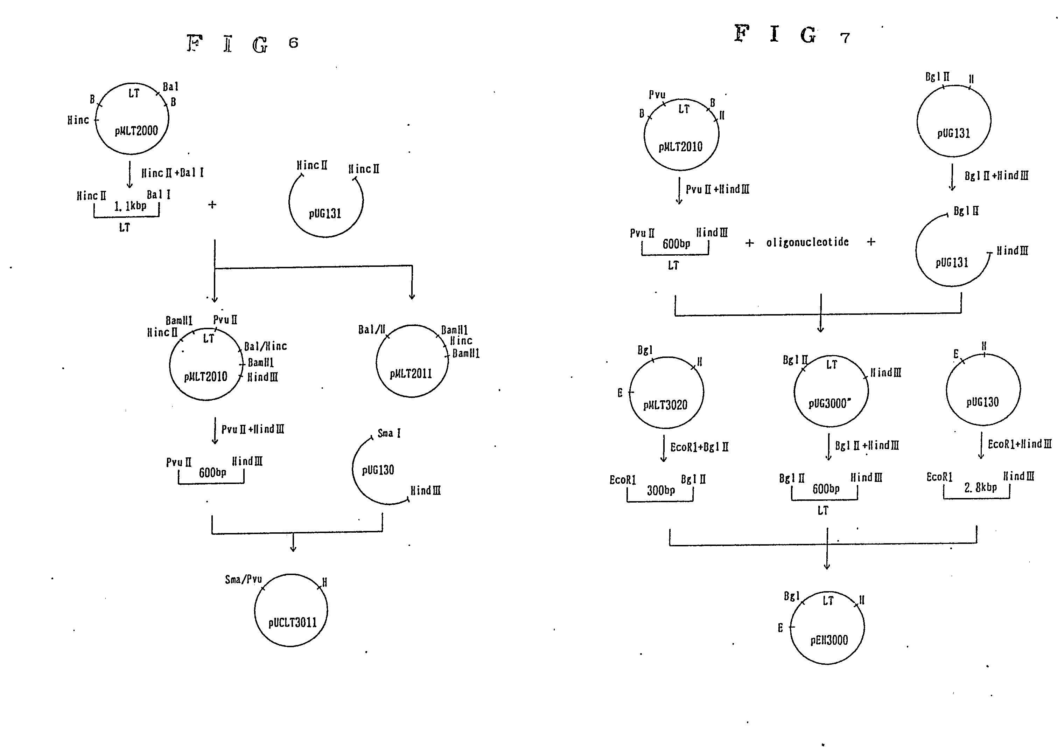

The flow diagram of this assembly is shown in Fig. 5.

5' AATTCAGATCTCATGCCCGC 3' NLT-15
3' GTCTAGAGTACGGG 5' NLT-16

### Example 5

Plasmid pMLT 2000 was digested with Hind II and Bal I to obtain a DNA fragment of about 800 bp. This fragment and another fragment of about 2.8 kbp prepared by the digestion of plasmid pUG131 with Hind II were enzymaticaly joined to form two plasmids, between which one which had been digested with BamHl to form a DNA fragment of about 800 bp and 2.8 kbp was named pMLT2010, while one which had been digested therewith to form a DNA fragment of about 3.5 kbp was named pMLT2011. Then, the plasmid pMLT2010 was digested with Pvu II and Hind III to obtain a DNA fragment of about 600 bp. This fragment and another DNA fragment of about 2.8 kbp prepared by the digestion of plasmid pUG130 with Sma I and Hind III were enzymatically joined (Plasmid No. pUCLT3011). The flow diagram of this assembly is shown in Fig. 6.

### Example 6

A DNA fragment of about 600 bp obtained by the digestion of plasmid pMLT 2010 with Pvu II and Hind III, the synthetic oligonucleotide phosphorylated at the 5 terminal which will be described, and another DNA fragment of about 2.8 kbp obtained by the digestion of plasmid pUG131 with Bgl II and Hind III were enzymatically joined (Plasmid No. pUG3000).

Then, the pUG3000 was digested with Bgl II and Hind III to obtain a DNA fragment of about 600 bp. This DNA fragment, another DNA fragment of about 300 bp prepared by the digestion of plasmid pUG130 with EcoRl and Hind III were enzymatically joined (Plasmid No. pEH3000). The flow diagram of this assembly is shown in Fig. 7.

5' GATCTGCATGCTCCCTGGTGTAGGCCTCACACCTTCAG 3'

3' ACGTACGAGGGACCACATCCGGAGTGTGGAAGTC 5'

### Example 7

A DNA fragment of about 600 bp prepared by the digestion of plasmid pMLT2010 with Pvu II and Hind III, the synthetic oligonucleotide phosphorylated at the 5 terminal which will be described, and another DNA fragment of about 7.3 kbp prepared by the digestion of M13tg130 double-stranded DNA (Pharmacia) with EcoRl and Hind III were enzymatically joined.

Synthetic oligonucleotide:

5' AATTCAGATCTCATGCTGCCGGGTGTAGGCCTGACTCCGTCAG 3'

3' GTCTAGAGTACGACGGCCCACATCCGGACTGAGGCAGTC 5'

E. coli JM103 was transformed with the obtained double-stranded DNA to obtain M13 phage plaque. Single-stranded DNA was prepared form the phage plaque and subjected to the in vitro mutagenesis using, as a primer, the following oligonucleotide:

5' GATACACCATGGCCTGGGAGCTGA 3'

Double-stranded DNA was prepared form the obtained mutant M13 phage and digested with Accl and Hind III to obtain a DNA fragment of about 300 bp. Separately, plasmid pMLT5001A was digested with EcoRl and Accl to obtain another DNA fragment of about 430 bp, while plasmid pUG130 was digested with EcoRl and Hind III to obtain another DNA fragment of about 2.8 kbp. The three DNA fragments were enzymatically joined (Plasmid No. pEH5153).

The flow diagram of this assembly is shown in Fig. 8.

### Example 8

DNA of about 5 kbp prepared by the digestion of plasmid pMLT5001 A with Bgl II and Sac II and DNA of about 2 kbp prepared by the digestion of pFGKI DNA with Bgl II and Sac II were ensymatically joined (Plasmid No. pLRM2000).

Then, pLRM2000 was digested with Bgl II and Sac II to obtain DNA of about 5 kbp. This DNA and the synthetic DNA which will be described were enzymatically joined. E. Coli RRI was transformed with the obtained plasmid to obtain a plasmid library. This library contained all kinds of LTs obtained by replacing the Lys present in the second position from the NH₂-terminal of pMLT5001A with all amino acids.

5' GATCTCATGNNNCCCGC 3' N = A, G, C, T
3' AGTACNNNGGG 5'

A highly active plasmid was selected from the library based on the cytotoxic effect on a mouse LP3 cell. The selected plasmid was digested with EcoRl and Hind III to obtain DNA of about 800 bp. Separately, M13mp110 double-stranded DNA was digested with EcoRl and Hind III to obtain another DNA. The both DNAs were enzymatically joined and transfected into JM103. Then, the DNA base sequence was determined by an ordinary method. The plasmids prepared by this process and the amino acid structure thereof are as follows:

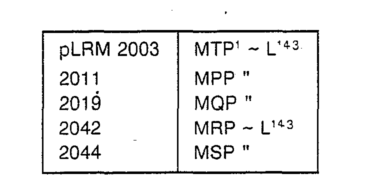

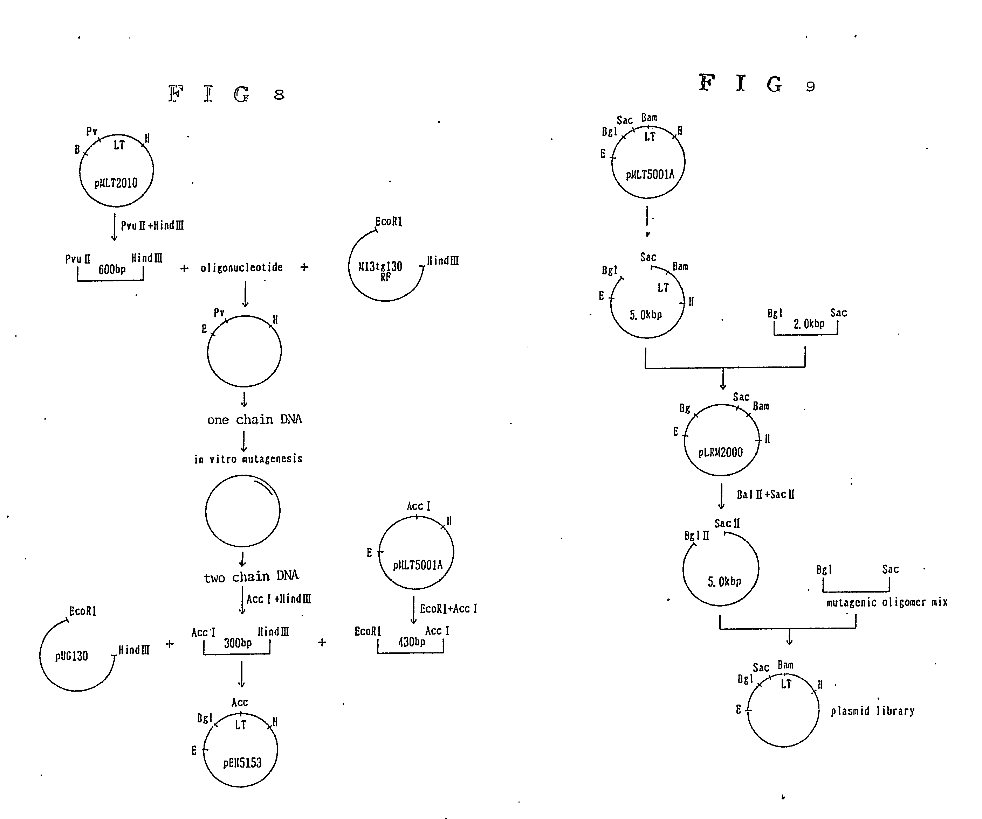

The flow diagram of this assembly of plasmid is shown in Fig. 9.

### Example 9

pMLT5001A was digested with EcoRl and Sac II to obtain a DNA fragment of about 300 bp. Separately, pMLT5001 was digested with BamHl and Hind III to obtain another DNA fragment of about 600 bp, while pFGKI was digested with BomHl and Soc II to obtain another DNA fragment of 1.6 kb. These fragments and another fragment prepared by the digestion of pUC12 with EcoRl and Hind III were enzymatically joined to obtain pLRM300.

The pLRM300 was digested with BamHl and Sac II to obtain DNA of about 3.5 kb. This DNA was joined to a synthetic oligonucleotide mixture: 
5'    GGCTNNNCTGATC          3'
3'  CGCCGANNNGACTAGNNNCTAG  5'
(N stands for A, G, C, T)
 E. coli RRI was transformed with the obtained plasmid to obtain a plasmid library.

This plasmid library was screened based on the cytotoxic effect on LP3 to select a plasmid wherein the amino acids His⁴, Ile⁶ and Gly⁷ of lymphotoxin are converted as follows:

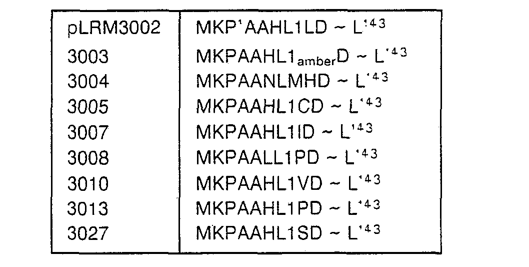

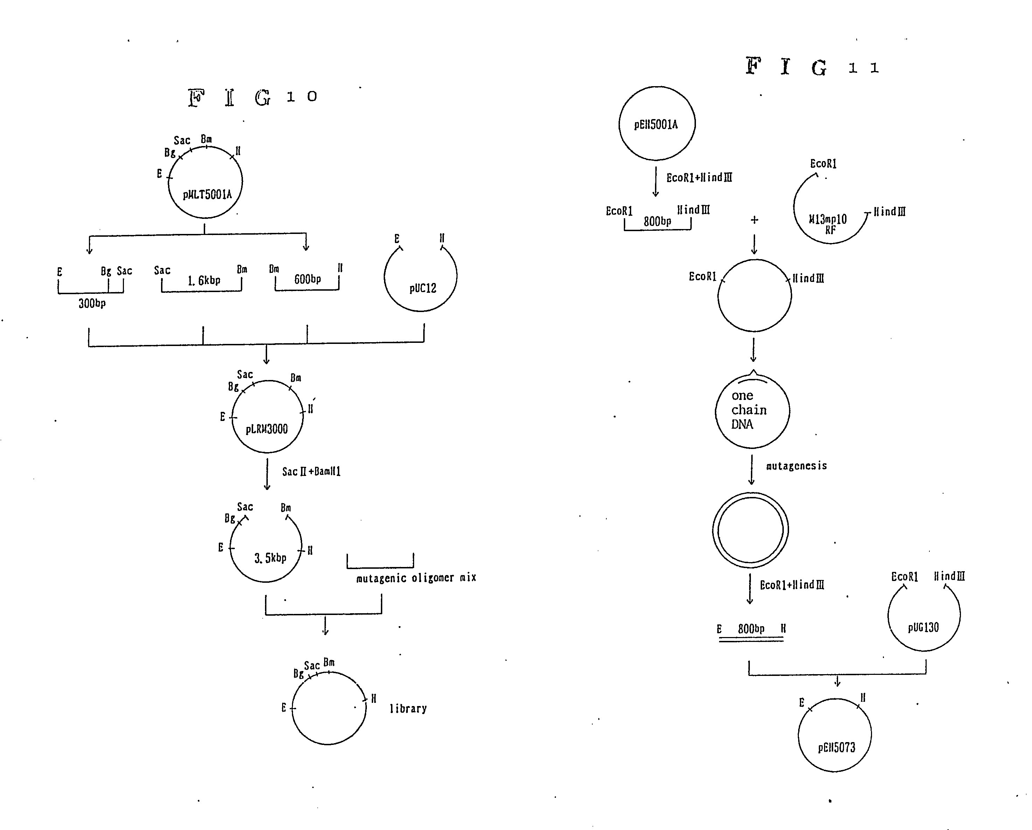

The flow diagram of this assembly is shown in Fig. 10.

### Example 10

pMLT5001A was digested with restriction enzymes EcoRI and Hind III to recover a fragment of about 800 bp from the resulting system by agarose gel electrophoresis. This fragment was enzymatically joined to DNA of about 7.2 bp prepared by the digestion of M13mP 10RF DNA with EcoRI and Hind III. E. coli JM103 was transformed with the obtained DNA to obtain a white plaque. Single-stranded DNA was prepared from the plaque and subjected to the mutagenesis using the following DNA: 3' GGGTCGTTAGTCTTAAGTGACG 5' as a primer in vitro.

Double-stranded DNA was prepared from the phage thus obtained and digested with EcoRl and Hind III to obtain a DNA fragment of about 800 bp. This fragment was enzymatically joined to another fragment of about 2.8 kbp obtained by the digestion of pUG130 with EcoRl and Hind III (Plasmid pEH5073). The flow diagram of this assembly is shown in Fig. 11.

### Example 11

The mutagenesis of the same single-stranded DNA as the one used in Example 10 was carried out by using the following synthetic DNA: 3' AAGGTCGACTGCTTACCTCTGGT 5' as a primer in vitro.

Double-stranded DNA was prepared from the phage prepared by the mutagenesis and digested with EcoRl and Hind III to obtain a DNA fragment of about 800 bp. This fragment was enzymatically joined to another fragment of about 2.8 kbp prepared by the digestion of pUG130 with EcoRl and Hind III (Plasmid pEH5177).

### Example 12

Plasmid pUC12 was digested with BamHl and Pst I to obtain a DNA fragment of about 2.8 kbp. This fragment was enzymatically joined to the following synthetic DNA phosphorylated at the 5 terminal to obtain plasmid pUC7692.

BamHI                                    Acc III
5'  GATCCGTCTACTCCCAGGTGGTTTTCT              3'
3'      GCAGATGAGGGTCCACCAAAAGAGGCC          5'
Acc III                                        xhoI
5'  CCGGAAAAGCTTACTCTCCGAACGCTACC            3'
3'      TTTTCGAATGAGAGGCTTGCGATGGAGCT        5'
xhoI                                           PstI
5'  TCGAGCCCGCTGTACCTGGCTCACGAGGTCCTGCA      3'
3'      CGGGCGACATGGACCGAGTGCTCCAGG          5'

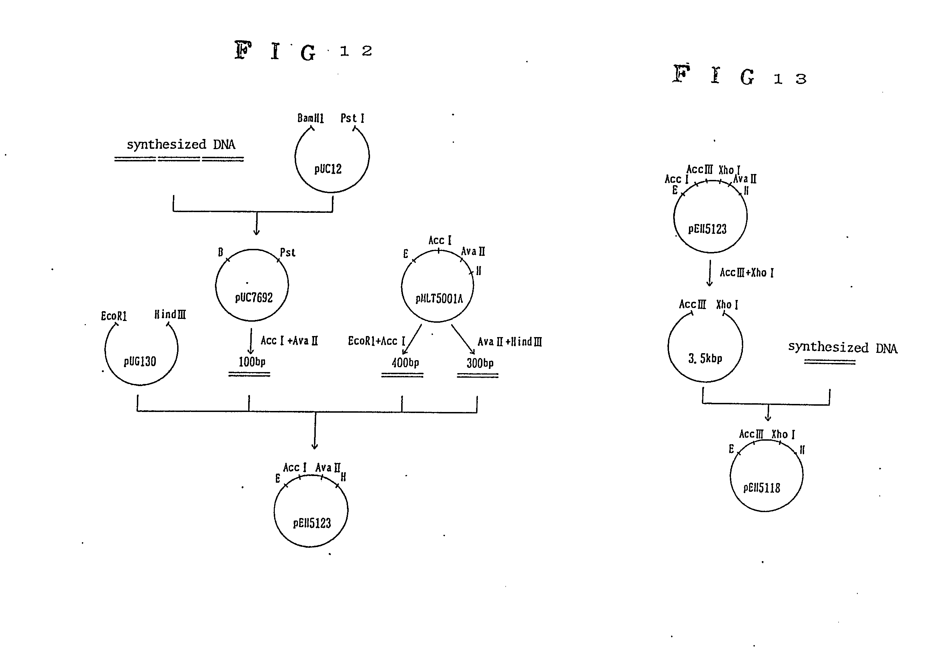

The plasmid pUC7692 was digested with Acc I and Ava II to recover a DNA fragment of about 100 bp from the resulting system by 7% polyacrylamide electrophoresis. Separately, plasmid pMLT5001' was digested with EcoRl and Acc I and with Ava II and Hind III to obtain a DNA fragment of about 450 bp and a DNA fragment of about 300 bp. respectively, while plasmid pUG130 was digested with EcoRl and Hind III to obtain a fragment of about 2.8 kbp. These four fragments were enzymatically joined (Plasmid pEH5123). The flow diagram of this assembly is shown in Fig. 12.

### Example 13

Plasmid pEH5123 was digested with Acc III and Xho I and treated with bacterial alkaline phosphorylase to carry out the dephosphorylation at the 5 terminal. A fragment of about 3.5 kbp was recovered from the resulting system by agarose electrophoresis and enzymatically joined to the following synthetic DNA (Plasmid pEH5118).

5'  CCGGAGGTGCTTACTCTCCGAACGCTACC      3'

3'      TCCACGAATGAGAGGCTTGCGATGGAGCT  5'

The flow diagram of this assembly is shown in Fig. 13.

### Example 14

Plasmid pEH5073 was digested with EcoRl and Sal I to obtain DNA of about 500 bp. Separately, plasmid pEH5073 was digested with BamHl and Sal I to obtain DNA of about 3.1 kbp. The both DNAs and the following synthetic DNA were enzymatically joined and transformed E. coli JM83 to obtain a plasmid library.

5'   GATCNGTNCAAGCAA      3'

3'        CANGTTCGTTTTA  5'

A plasmid having an antitumor activity was selected from this library to determine the structure thereof. The obtained plasmid had the following structure:

pEH5071-1 | MKAAHLIGD RF QN ∿ L^143
-2 | " PF "
-7 | " LF "
-13 | " RC "
-19 | " QF "
-24 | " RS "

The flow diagram of this assembly is shown in Fig. 14.

### Example 15

pMLT5001A was digested with restriction enzymes EcoRl and Hind III to recover a lymphotoxin- containing fragment of about 800 bp. This fragment was inserted into the EcoRl and Hind III sites of M13mp 10RF DNA to isolate single-stranded DNA. The mutagenesis of this single-stranded DNA was carried out by using the following synthetic DNA. 
Xba I
5' GGAGAGAATTTCTAGACAAGGAG 3'
 as a primer in vitro.

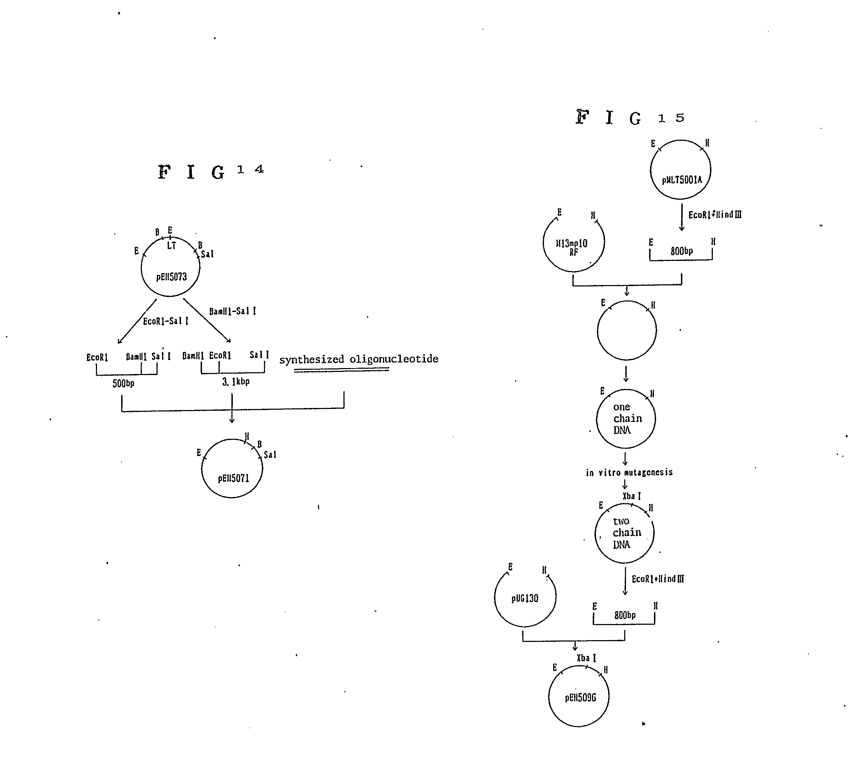

Double-stranded DNA was prepared from the plaque selected by plaque hybridization and digested with EcoRl and Hind III to obtain a DNA fragment of about 800 bp. This fragment was enzymatically joined to another fragment of about 2.8 kbp prepared by the digestion of pUG130 with EcoRl and Hind III (Plasmid No. pEH5096). The flow diagram of this assembly is shown in Fig. 15.

### Example 16

A DNA of 3741 bp was obtained by cleaving plasmid pBR322 with Baℓ I and Pvu II. Subsequently, pBR322 d-rop consisting of 3741 bp was obtained by enzymatic linking. The pBR322 d-rop as decribed above was a plasmid deprived of the fuction of repressor of primer which controlled the replication of plasmid DNA and a high copied number of plasmid could be expected from, compared with that of pBR322.

Then, a DNA fragment of about 1800 bp obtained by cleaving plasmid pBR322 d-rop with EcoR I and Pvu I and a DNA fragment of about 3100 bp obtained by cleaving plasmid pMLT5011 with EcoR I and Pvu I were linked ( Plasmid No. pBR5011 )

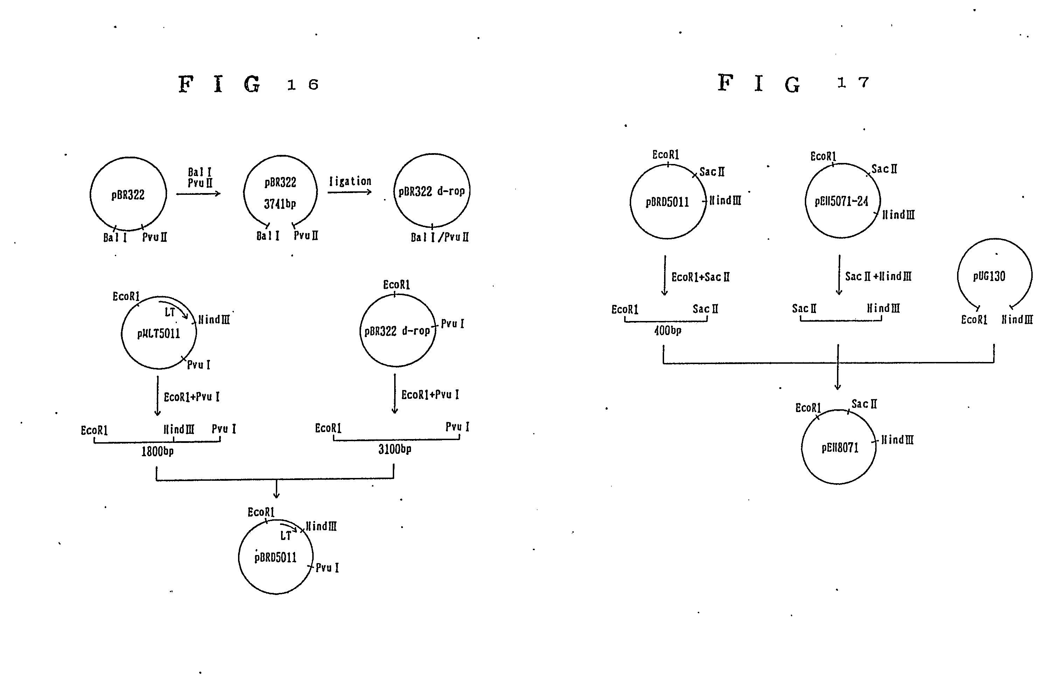

The flow diagram of the assembly is shown in Fig.16.

In addition, a DNA of about 450 bp obtained by cleaving pEH5071-24 with Sac II and Hind III was enzymatically inserted into the site of EcoR I and Hind III of plasmid pUG130, together with a DNA fragment of about 400 bp obtained by cleaving pBRD5011 with EcoR I and Sac II ( Plasmid No. pEH8071 ).

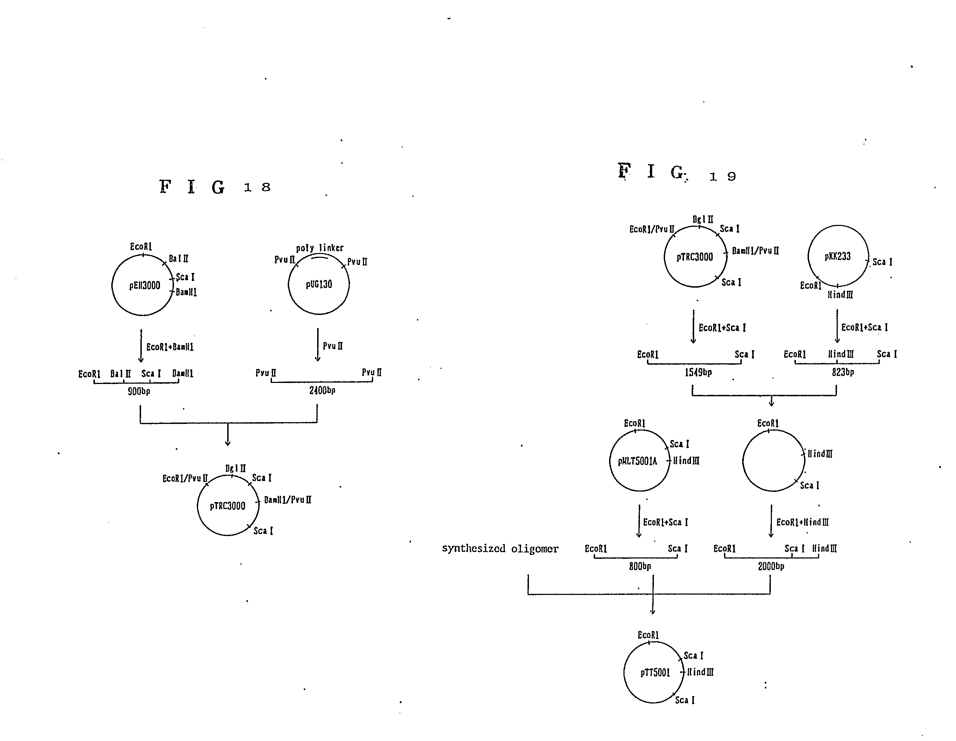

The flow diagram of the assembly is shown in Fig. 17.

The modification of plasmid pEH8071 was aimed to improve the stability of mRNA.of E. coli and to prevent mutant E. coli from skipping the translation of the terminal codon because the plasmid contains the terminal codon TAG and the untranslated region of about 100 bp derived from natural lymphotoxin cDNA.

That is, firstly, a DNA of about 2400 bp obtained by cleaving pUG130 with Pvu II and a DNA of about 900 bp obtained by cleaving pEH300 with EcoR I and BamHl were linked and a plasmid where lymphotoxin cDNA was inserted thereinto at the same direction of ampocillin cDNA was selected ( Plasmid No. pTRC3000 ).

The flow diagram of the assembly is shown in Fig. 18.

Secondly, a DNA fragment of 1549 bp obtained by cleaving pTRC3000 with EcoR I and Sca I and a DNA fragment of 823 bp obtained by cleaving pKK233-2 ( Pharmacia Co.) with EcoR I and Sca I were linked. A DNA fragment of about 2000 bp obtained by cleaving said plasmid with EcoR I and Hind II1, a DNA fragment of about 800 bp obtained by cleaving pMLT5001A with EcoR I and Sca I and the following synthetic DNA were enzymatically linked ( Plasmid No. pTT5001 ).

The flow diagram of the assembly is shown in Fig.19.

A DNA fragment of about 600 bp obtained by cleaving pEH8071 with EcoR I and Pst I, a DNA fragment of about 1700 bp obtained by cleaving pTT5001 with Pst I and Pvu I and a DNA fragment of about 3100 bp obtained by cleaving pBR322 d-rop with EcoR I and Pvu I were enzymatically linked ( Plasmid No. pBRD8071 ).

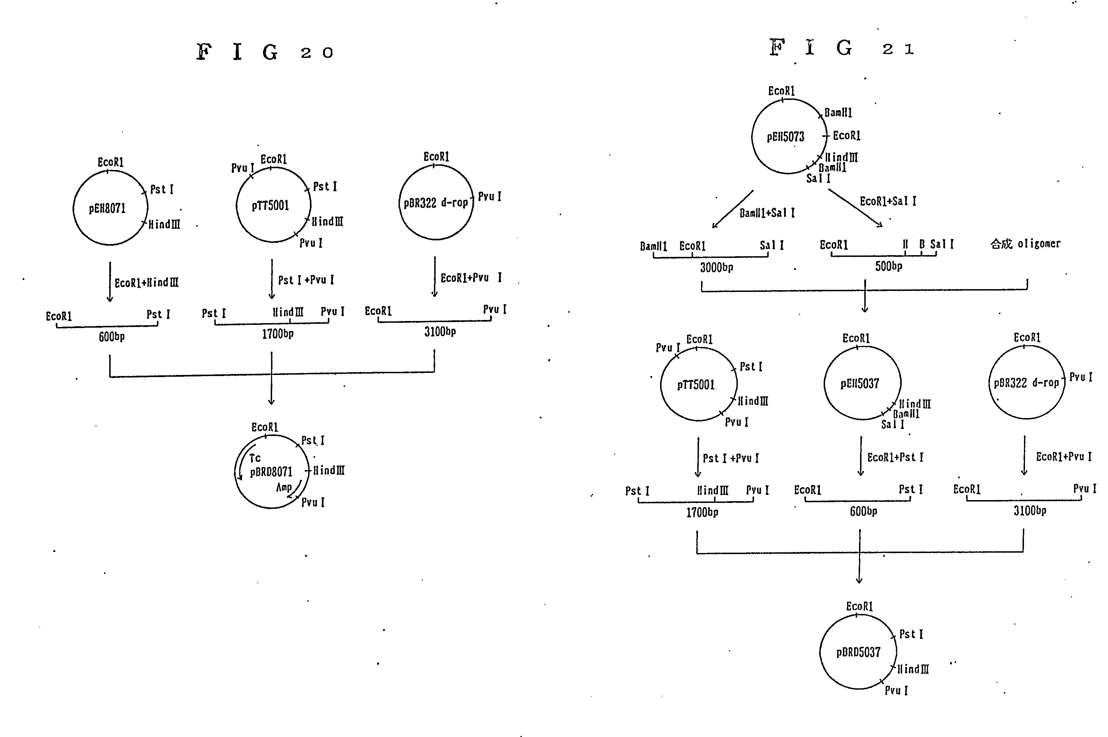

The flow diagram of the assembly is shown in Fig.20.

### Example 17

A DNA fragment of about 3000 bp obtained by cleaving pEH5073 plasmid with BamHl and Sal I, a DNA fragment of about 500 bp obtained by cleaving pEH5073 with EcoR I and Sal I and the following synthetic DNA were enzymatically linked.

5'    GATGCAGCAAGCAA    3'    37A -15A

3'    GGTCGTTCGTTTTAA    5'    37A -15B

A plasmid was recovered from E. coli. which was tranformed by the plasmid prepared as described above and an analysis of the DNA structure thereof demonstrated that C changed into T at the fifth site from 5' terminal of 37A-15A ( Plasmid No. pEH5037 ). A DNA fragment of about 600 bp obtained by cleaving pEH5037 with EcoR I and Pst I. a DNA fragment of about 1700 bp obtained by cleaving pTT5001 with Pst I and Pvu I and a DNA fragment of about 3100 bp obtained by cleaving pBR322 d-rop with EcoR I and Pvu I were enzymaticaly linked ( Plasmid No. pBRD5037 ).

The flow diagram of the assembly is shown in Fig.21.

. A DNA fragment of about 450 bp obtained by cleaving plasmid pEH5037 plasmid with Sac II and Hind III, a DNA fragment of about 400 bp obtained by cleaving pBRD5011 with EcoR I and Sac II were enzymatically inserted into the site of EcoR I and Hind III of pUG130 ( Plasmid No. pEH8037 ).

Subsequently, a DNA fragment of about 600 bp obtained by cleaving pEH8037 with EcoR I and Pst I, a DNA fragment of about 1700 bp obtained by cleaving pTT5001 with Pst I and Pvu I and a DNA fragment of about 3100 bp obtained by cleaving pBR322 d-rop with EcoR I and Pvu I were enzymatically linked ( Plasmid No. pBRD8037 ).

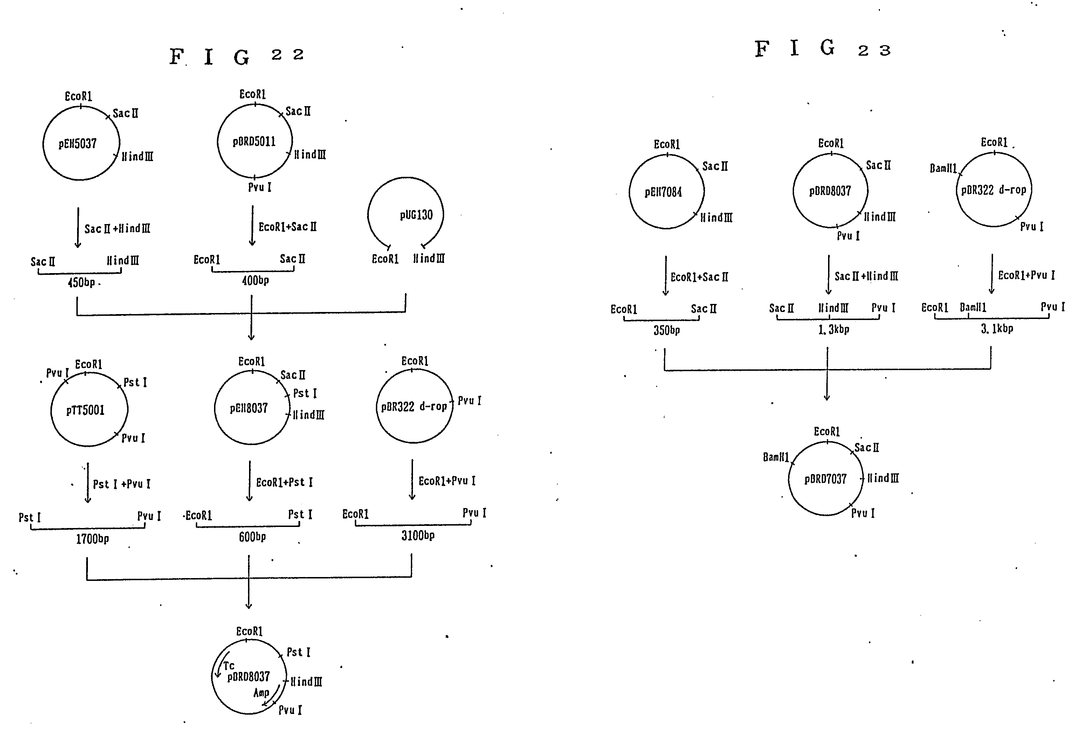

The flow diagram of the assembly is shown in Fig. 22.

### Example 19

A DNA fragment of about 350 bp obtained by cleaving plasmid pEH7084 with EcoR I and Sac II, a DNA fragment of about 1.3 kbp obtained by cleaving pBRD5037 with Sac II and PVu I and a DNA fragment of about 3.1 kbp obtained by cleaving pBR322 d-rop with EcoR I and Pvu I, all three DNA fragments were enzymatically linked ( Plasmid No. pBRD7037 ).

The flow diagram of the assembly is shown in Fig. 23.

### Example 20

A DNA fragment of about 350 bp obtained by cleaving plasmid pEH7084 with EcoR I and Sac II, a DNA fragment of about 1.3 kbp obtained by cleaving pBRD8071 with Sac II and PVU I and a DNA fragment of about 3.1 kbp obtained by cleaving pBR322 d-rop with EcoR I and Pvu I, all three DNA fragments were enzymatically linked ( Plasmid No. pBRD7071 ).

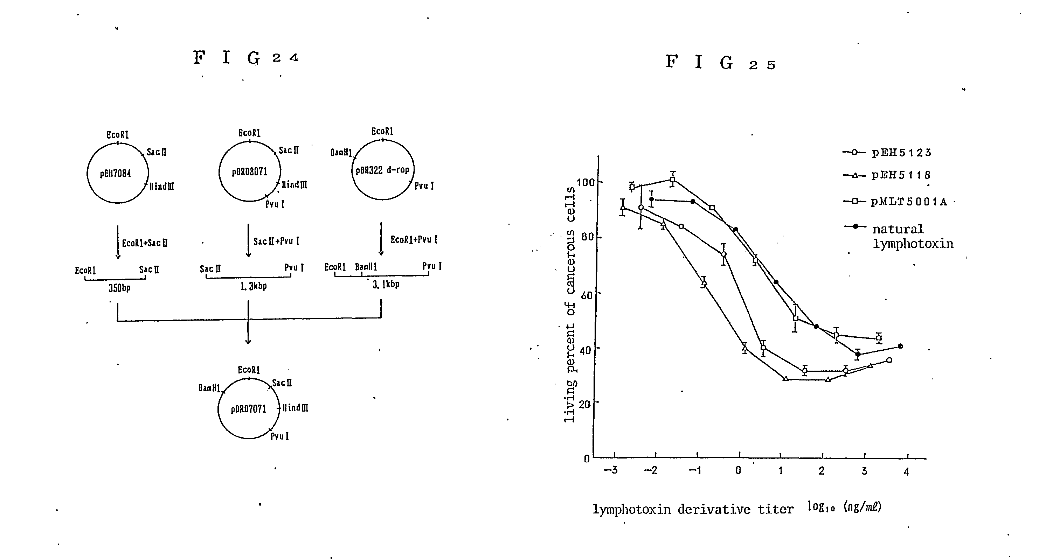

The flow diagram of the assembly is shown in Fig.24.

### Example 21

Escherichia coli was transformed with the plasmid prepared in the above Example according to an ordinary method and cultured. followed by the purification of the objective substance. An example wherein pUCLT3011 is used will be described.

Recombinant E. coli JM83,pUCLT3011 was shake-cultured on 2ℓ of an LB medium (1% bactotrypsin, 0.5% yeast extract, and 0.5% NaCI) containing 100 µg/ml of ampicillin at 37°C for 14 hours and centrifugally collected. The obtained wet residue was suspended in about ten times as much 10mM PBS (pH 7.4), disintegrated with a sonicator under cooling and centrifuged at the rate of 20,000 rpm for 30 minutes to obtain 28 ml of a supernatant fraction (crude extract). This crude extract exhibited an LT activity of 7.4 x 10⁶ unitmg protein. The crude extract was placed on a gel filtration medium Sephacryl S-200 which had been sufficiently equilibrated with 200 mM phosphate buffer (pH 6.8) and eluted with Sephacryl-200. The obtained fraction (100 ml) having an LT activity was placed on a pigment adsorption affinity carrier Procion red agarose which had been sufficiently equilibrated with 200 mM phosphate buffer (pH 6.8), sufficiently washed with the same equilibration buffer as the one used above and eluted with a solution of arginine in 100 mM Tris buffer (pH 9.4) with a straight concentration gradient of 0 mM to 400 mM. A fraction having an LT activity was obtained at an arginine concentration of 180 to 220 mM. Then, the fraction (12.5 ml) having an LT activity was diluted with 100 mM Tris buffer (pH 9.4)/2 M Na₂SO₄, 0.2 M Arg to make up to a total volume of 25 ml.

The diluted fraction was subjected to high-performance liquid chromatography with TSK gel Ether-5PW (7.5 x 75 mm). Namely, the fraction (2.0 ml) having an LT activity and equilibrated with 1 M Na₂SO₄ was placed on TSK gel Ether 5PW which had been sufficiently equilibrated with 100 mM Tris buffer (pH 9.4);1 M Na₂SO₄ and eluted with a straight concentration gradient of from 100 mM Tris buffer (pH 9.4)/1 M Na₂SO₄ to 100 mM Tris buffer (pH 9.4)_{/}0.2 M Arg. A fraction having an LT activity was obtained at a Na₂SO₄ concentration of 220 to 180 mM. This fraction was used as a final preparation. The preparation exhibited a specific LT activity of 2.7 x 10⁸ unit_{/}mg protein. which means that the crude extract was purified about 36-fold.

### Example 22

Recombinant E. coli JM 83₁pMLT 5001 A was cultured and treated according to the same procedure as that of Example 16 to obtain 21 ml of a crude extract. This extract was purified in a similar manner to the one of Example 16 to obtain a single substance having a purity of 95% or above as determined by HPLC. The NH₂-terminal sequence and analyzed amino acid composition of pMLT5000A are shown in Table 1. The specific activity as calculated from the analyzed amino acid composition was 3.4 x 10⁸ U/mg.

The term "1 U" refers to the amount of pMLT5001A which can necrotize 50% of mouse LP3 cells.

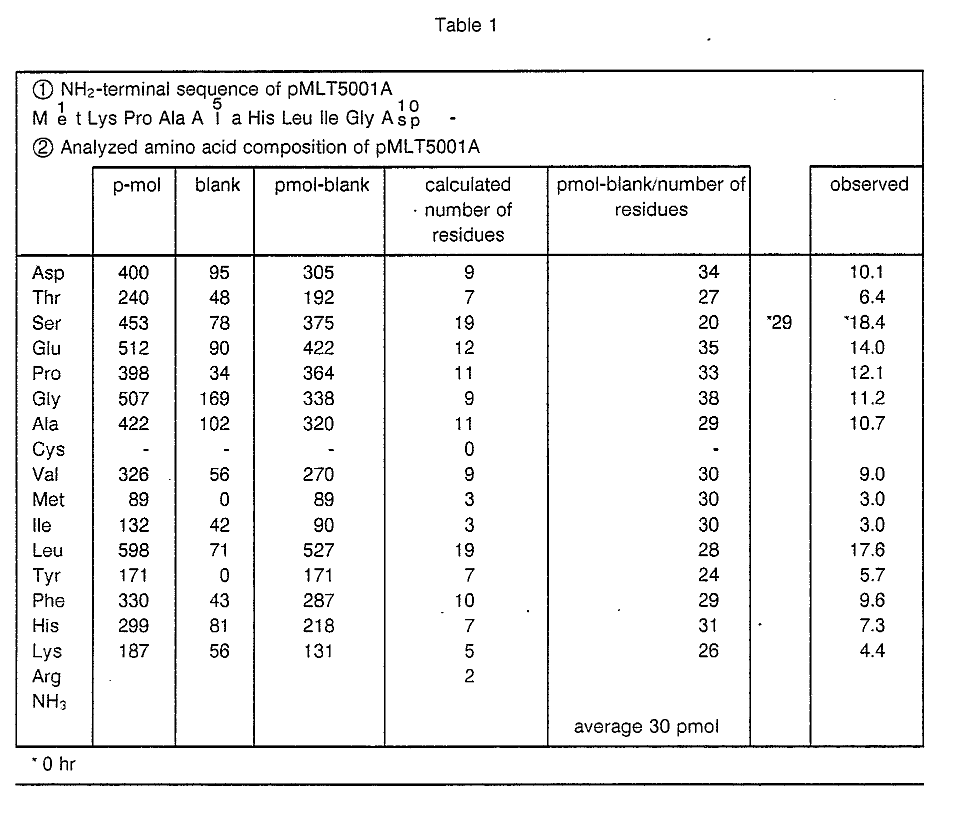

### Example 23

Recombinant E. coli ( RR1 pBRD5037 ) was cultured in the presence of ampicillin ( 100 µg/mℓ) in 400 ml of LB medium ( Bacto tryptone 10g/ℓ. NaCl 5g.ℓ, yeast extract 10g/ℓ) at 37 °C for 15 hours. Then, the resulting system was transferred into 10 t of M9CA medium ( 1 % of casamino acid, 1 % of lactose Na₂HPO₄ 6g.t . KH₂PO₄ 3g/ℓ, NaCl 0.5g/ℓ, NH₄Cl 1g/ ℓ ,FeSO₄ 7H₂0 0.5 mg/ℓ, 2mM MgSO₄, 0.1mM CaCl₂ and cultured under continuous shaking at 37 C. After 15 hours, 500 ml of casamino acid ( 20 %) was added and cultured for another 25 hours before collecting the cells by centrifuge and suspended the collected cells in 100 ml of PBS ( Dulbecco ). After this suspension was disintegrated with a sonicator in an ice -cold condition and centrifuged at 20000 rpm for 30 minutes, 132 ml of the supernatant was obtained. The crude extract was purified by the following method.

The crude extract ( 2.8 x 10¹⁰ units, ml ) was applied to a Sephadex S - 200 column ( φ 133 x 900 mm which was sufficiently equilibrated with 0.2 M phosphate buffer ( pH 6.8 ). And then, the fraction exhibiting LT activity was collected and applied to a Sepharose column ( φ 55 x 210 mm ) conjugated with the monoclonal anti-LT antibodies, followed by sufficient wash by 0.2 M phosphate buffer ( pH followed by sufficient wash by 0.2 M phosphate buffer ( pH 6.8) and elution with 0.2 M phosphate buffer ( pH 6.8). After the obtained LT fraction of 232 ml was concentrated up to 82 ml with an instrument of ultrafiltration, the fraction was desalted by Sephadex. g - 25' ( φ 55 x 210 mm ) which was sufficiently equilibrated with 20 mM phosphate buffer (pH 6.8). The purity of the LT derivative obtained in this procedure which was determined by SDS - PAGE analysis was 98 % or more.

### Example 24

After recombinant E. coli( RR1ipBRD7037 ) was cultured at the scale of 10 ℓ in a similar procedure as described in Example 23 and collected after disintegration, a crude extract of 150 m ℓ { ( 1.1 x 10'° units ml ) was obtained. This crude extract was applied in order to the column of Sephadex S -200, that of Sepharose conjugated with the monoclonal anti - LT antibodies and that of Sephadex G -25 and the resulting purified protein which exhibited a purity of 98 % or more by SDS -PAGE analysis was obtained.

### Example 25

After recombinant E. coli ( RR1 pBRD8037 ) was cultured at the scale of 20 ℓ in a similar procedure as described in Example 23 and collected before disintegration, a crude extract of 160 mt ( 4.8 x 10¹⁰ units m t ) was obtained. This crude extract was applied in order to the column of Sephadex S -200, that of Sepharose conjugated with the monoclonal anti-LT antibodies and that of Sephadex G -25 as described in Example 2 and the resulting purified protein which exhibited a purity of 98 % or more by SDS -PAGE analysis was obtained.

### Example 26

### Preparation of mRNA

Daudi cells (2 x 10⁸) was suspended in 10 ml of a solution containing 4M guanidinium thiocyanate, 0.5% of sodium N-laurolysarcosine. 25 mM sodium citrate (pH 7.0), 0.1 M mercaptoethanol and 0.1% of antiform (sigma). The obtained suspension was homogenized with a Politron. The obtained homogenate was layered on an 5.7 M aqueous solution of cesium chloride containing 0.1 M EDTA. The resulting system was centrifuged with an ultracentrifuge (SRP 28 A rotor: a product of Hitachi) at the rate of 26000 rpm at 20°C for 23 hours. The obtained RNA precipitate was dissolved in 10 ml of 10 mM Tris-HCI (pH 7.4), followed by the addition of 2.5 times as much ethanol. The resulting mixture was allowed to stand at -70 C for one hour and centrifuged to form an RNA precipitate. This precipitate was dissolved in 4.14 ml of 10 mM Tris-HCI (pH 7.4), followed by the addition of 2 ml of 4M potassium chloride. The obtained RNA solution was passed through an oligo(T)cellulose column (₀2 x 15 cm). The column was sufficiently washed with 0.5 M potassium chloride containing 10 mM Tris-HCL (pH 7.4). Then a 10 mM aqueous solution of Tris-HCI (pH 7.4) was passed through the column to elute mRNA.

### Purification of mRNA

500 µg of the mRNA prepared from Daudi cells was layered on 15 ml of a solution containing 50 mM Tris-HCI (pH 7.4), 0.2 M NaCl and 1 mM EDTA and having a sucrose concentration gradient of from 10 to 28%. The resulting system was centrifuged with an ultra-centrifuge (SRP 28 A rotor: a product of Hitachi) at the rate of 26000 rpm at 20° C for 16 hours and fractionated to obtain fractions each of 50 µl. Ethanol was added to each fraction to form a mRNA precipitate. This precipitate of each fraction was dissolved in sterilized water to obtain a solution. The mRNA concentration of the solution was adjusted to 1 µgµl.

### Screening of mRNA

Serum gonadotrophin (200 U) (Peamex: a product of Sankyo) was injected into a female Xenopus of one year or older. After the lapse of one night, the oocyte was taken out and preserved in a modified barth saline medium (MBS). The mRNA of each fraction prepared above was injected into egg in an amount of 50 to 100 ng/egg and cultured in MBS containing 1 mM phenyl-methylsulfonyl fluoride (PMSF) at 25°C for 36 hours. After the removal of a supernatant, the oocyte was homogenized and extracted with MBS containing 1 mM PMSF to obtain a mRNA sample of each fraction. Each sample was examined for lymphotoxin, activity and screened to obtain a mRNA fraction exhibiting lymphotoxin activity.

### Preparation of cDNA library

. 5 ug of the mRNA fraction exhibiting lymphotoxin activity contained in the mRNA expression system prepared by the use of Xenopus oocyte was dissolved in 7 µl of 5 mM Tris-HCI (pH 8.3) and incubated at 65 C for 5 minutes, followed by the addition of 1.4 u.g of pcDV-1 DNA (Pharmacia): The obtained mixture was reacted under the following conditions: 50 mM Tris-HCI pH 8.3, 8 mM MgCl₂, 30 mM KCI, 0.3 mM DTT, 2 mM dNTP, reverse transcriptease 5U reaction volume 20 µl.

After the reaction had been carried out at 37 C for 60 minutes, the reaction was stopped by the addition of 2 µl of 0.25 M EDTA. The reaction mixture was treated with phenol/chloroform, followed by the addition of 20 µl of 4 M NH₄OAc and 80 µl of EtOH. The obtained mixture was allowed to stand at -70° C and centrifuged to remove the supernatant. The residue was repeatedly subjected to the same treatment as the one described above. The obtained precipitate was washed with 75% aqueous EtOH, dried and adjusted to a reaction solution of 10 mM sodium cacodylate, 2.5 mM HEPES (pH 7.6), 0.1 mM dCTP, and 0.2 µg/µl poly (A). Finally, the volume of the solution was adjusted to 20 µl by the addition of 10 U of terminal deoxynucleotidyl transferase (TdT). The reaction was carried out at 37 C for 10 minutes and stopped by the addition of 1 µl of 0.25 M EDTA. The reaction mixture was post-treated by an ordinary method.

The obtained DNA precipitate was dissolved in 11 µl of a 10 mM Tris-HCI solution (pH 7.4) containing 8 mM MgCl₂, 6 mM mercaptoethanol and 10 U of Hind III to carry out the reaction at 37° C for 2 hours. The reaction was stopped by the addition of 1 µl of 0.25 M EDTA. The reaction mixture was post-treated by an ordinary method and dissolved in a mixture comprising 10 µl of a solution of 1 mM EDTA in 10 mM Tris-HCI (pH 7.4) and 3 µl of EtOH.

77 ng of pL-1 DNA (Pharmacia) was added to 11 µl of the DNA solution to obtain a mixture. A solution containing 10 mM Tris-HCI (pH 7.4), 1 mM EDTA and 0.1 M NaCl was added to the mixture to form a solution 120 µl). This solution was allowed to stand at 65 °C for 5 minutes and at 42° C for 30 minutes. Then. the following components were added to the resulting solution under cooling with ice to make up to a total volume of 1.2 ml:
20 mM Tris-HCI (pH 7.5). 4 mM MgCl₂, 10 mM (NH₄)₂SO₄, 0.1 M KCI. 0.1 mMβ-NAD, 50 µg/mℓ bovine serum albumin and 1 µg of E. coli ligase.

The reaction was carried out at 12° C overnight, followed by the addition of 10 µl of 4 mM dNTP, 10 ℓ of 20 mM 3-NAD, 1 µg of E. coli ligase. 17.5 U of E. coli DNA polymerase I and 120 U of RNase H. The obtained mixture was reacted at 12°C for one hour and then at 25°C for another one hour to obtain a reaction solution. This solution was used in the transformation of E. coli ₓ1776.

That is. E. coli ₓ1776 was cultured in a ₓ1776 medium (Maniatis et al.. Molecular cloning) at 37° C, until the absorbance thereof reached 0.2. The cultured E. coli χ1776 was gathered to prepare a competent cell according to Maniati's method. The above DNA was added to the competent cell to transform the E. coli x1776. Thus. a cDNA library was obtained.

### Screening of cDNA

In order to select a plasmid containing cDNA coding lymphotoxin from the above cDNA library, DNA corresponding to the eight-amino acid sequence of the C-terminal of lymphotoxin was synthesized and the colony hybridization was carried out by using the DNA as a probe.

The structure of the synthesized probe is GTC TTC TTT GGA GCC TTC GCT CTG. The probe was synthesized with an automatic solid phase synthesizer (Applied Biosystem) and purified by HPLC. The hybridization was carried out according to Wallace's method (Nucleic Acids Res, 9, 879 (1981)). Among the about 100000 cDNA libraries obtained. 60 libraries were positive colonies. Plasmids were prepared from 3 colonies out of the positive colonies and examined for base sequence. Only one of the plasmids contained lymphotoxin cDNA. The base sequence of the amino acid coding region of the obtained lymphotoxin cDNA is shown in Fig. 1.

### Assembly of expressive vector

The plasmid containing the lymphotoxin cDNA prepared above was digested with BamHl to obtain a fragment about 1.1 kbp) containing the lymphotoxin cDNA. This fragment was inserted into the BamHl site. of pUG 131 (a plasmid obtained by replacing the poly linker region of pUC 13 (Pharmacia) with that of M13tgl 31 (Pharmacia)) (Plasmid No. pMLT 2000). The obtained plasmid was digested with Hinc II and Bal I to obtain a fragment about 1.1 kbp). Separately, pKK233-2 (Pharmacia) was digested with Nco I and treated with Klenow enzyme to obtain a vector. The above fragment (1.1 kbp) was inserted into the vector (Plasmid No. pMLT 2100). The pKK 233-2 contains a trs promoter (one obtained by improving a tac promoter) and can'be expressed by the induction of IPTG. Then, the pMLT 2100 was digested with EcoRl and Hind III to obtain a fragment (about 1.5 kbp) containing the lymphotoxin cDNA. This fragment was inserted into M13 mp10 double-stranded DNA (Pharmacia), followed by the transfection thereof into E. coli JM103. According to an ordinary method, single-stranded DNA was prepared to carry out the in vitro metagenesis thereof by using the following synthetic oligonucleotide:

### GCT TGC TGG GAT CCA TGA GAT CTG TTT CCT

The site-directed mutagenesis was fundamentally carried out by Zoller's method (see Method in Enzymology, 100, 468 (1983)). Namely, 1 u.g of the template DNA (single-stranded DNA), 2 µl of 5 x annealing buffer (50 mM Tris-HCI, pH 8.0, 25 mM MgC1₂), 8 µl of B buffer (0.2 M Tris-HCI pH 7.5, 0.1 M MgCl₂, 0.1 M DTT), 4 ul of 2.5 mM dNTP, 2 µl of 5 mM ATP, 1 µl of distilled water, 5 U of T4 DNA ligase, 5 U of DNA polymerase I and Klenow fragment were added to 20 p mol of the above synthetic oligonucleotide labeled with nonradioactive phophoric acid at the end to make up to a total volume of 20 µl. The obtained mixture was reacted at 14° C for 3 hours and transformed E. coli JM103 (see Kramer et al., Cell 38, 879 (1984)).

The transformed bacteria were mixed with E. coli JM103. The obtained mixture was spreaded on an agar medium and cultured at 37 C overnight to form a plaque.

This plaque was transferred to nitrocellulose (Grunstein et al., PNAS 72, 3961 (1975)) and subjected to the hybridization test with a ³²P-labeled synthetic oligonucleotide to select a phage plaque wherein mutagenesis was induced. A double-stranded DNA was prepared from the phage plaque thus selected and digested with EcoRl and Hind III to obtain a DNA fragment. This fragment was inserted into a plasmid prepared by the digestion of pKK233-2 with EcoRl and Hind III (Plasmid No. pMLT 3020).

The pMLT 3020 is an expressive vector which initiates the synthesis of protein from the ATG on the downstream side of the Shine-Dargaro sequence of the trc promoter with eleven intervening nucleotides, which is digested with Bgl II to permit the insertion of an arbitrary gene into a position on the downstream side of the promoter and which is digested with BamHl to permit a gene into a position on the downstream side of the initiator codon ATG. The pMLT 3020 can code the polypeptide corresponding to the one obtained by removing the seven amino acid residues from the NH₂-terminal of the polypeptide of the present invention. The DNA sequence around the NH₂-terminal is as follows:

Trc promoter region

GTTGACAATTAATCAACCGGCTCGTATAATGTGTGGAATTG

SD Bgl II
TGAGCGGATAACAATTTCACACAGGAAACAGATCTC

Met Asp8 Pro9 Ser10 ...
ATG GAT CCC AGC ...
BamHI

The flow diagram of the assembly of pMLT 3020 is shown in Fig. 2.

pMLT3020 was digested with BamHl and Hind III to recover a DNA fragment of about 500 bp by agarose gel electrophoresis. Separately, the oligonucleotide which will be described was synthesized and phosphorylated at the 5 terminal with polynucleotide kinase (NEB) and ATP. The phosphorylated oligonucleotide, the above DNA fragment of 500 bp and a vector obtained by the digestion of pUG130 with EcoRl and Hind III were enzymatically joined (Plasmid No. pUG5001).

5' AATTCAGATCTCATGAAA 3' NLT-5
3' GTCTAGAGTACTTTGGGCGC 5' NLT-6
5' CCCGCGGCTCATCTGATCGGG 3' NLT-1
3' CGAGTAGACTAGCCCCTAG 5' NLT-2

After the termination of the reaction, E. coli JM83 was transformed according to an ordinary process to select a white colony on an X-Gal-containing agar plate. Then, the plasmid pUG5001 was digested with Bgl II and Hind III to recover a DNA fragment of about 500 bp by agarose gel electrophoresis. This fragment was enzymatically joined to DNA (about 4 kbp) prepared by the digestion of pMLT3020 with Bgl II and Hind III (Plasmid No. pMLT5001A). The flow diagram of this assembly is shown in Fig. 3.

Plasmid pUC12 was digested with BamHl and Pst I to obtain a DNA fragment of about 2.8 kbp. This fragment was enzymatically joined to the following synthetic DNA phosphorylated at the 5 terminal to obtain plasmid pUC7692.

BamHI                                    Acc III
5'  GATCCGTCTACTCCCAGGTGGTTTTCT                3'
3'      GCAGATGAGGGTCCACCAAAAGAGGCC            5'
Acc III                                        xhoI
5'  CCGGAAAAGCTTACTCTCCGAACGCTACC              3'
3'      TTTTCGAATGAGAGGCTTGCGATGGAGCT          5'
xhoI                                           PstI
5'  TCGAGCCCGCTGTACCTGGCTCACGAGGTCCTGCA        3'
3'      CGGGCGACATGGACCGAGTGCTCCAGG            5'

The plasmid pUC7692 was digested with Acc I and Ava II to recover a DNA fragment of about 100 bp from 500 bp by agarose gel electrophoresis. this fragment was enzymatically joined to DNA (about 4 kbp) prepared by the digestion of pMLT3020 with Bgl II and Hind III (Plasmid No. pMLT5001A). The flow diagram of this assembly is shown in Fig. 3.

Plasmid pUC12 was digested with BamHl and Pst I to obtain a DNA fragment of about 2.8 kbp. This fragment was enzymatically joined to the following synthetic DNA phosphorylated at the 5 terminal to obtain plasmid pUC7692.

BamHI                                    Acc III
5'  GATCCGTCTACTCCCAGGTGGTTTTCT                3'
3'      GCAGATGAGGGTCCACCAAAAGAGGCC            5'
Acc III                                        xhoI
5'  CCGGAAAAGCTTACTCTCCGAACGCTACC              3'
3'      TTTTCGAATGAGAGGCTTGCGATGGAGCT          5'
xhoI                                           PstI
5'  TCGAGCCCGCTGTACCTGGCTCACGAGGTCCTGCA        3'
3'      CGGGCGACATGGACCGAGTGCTCCAGG            5'

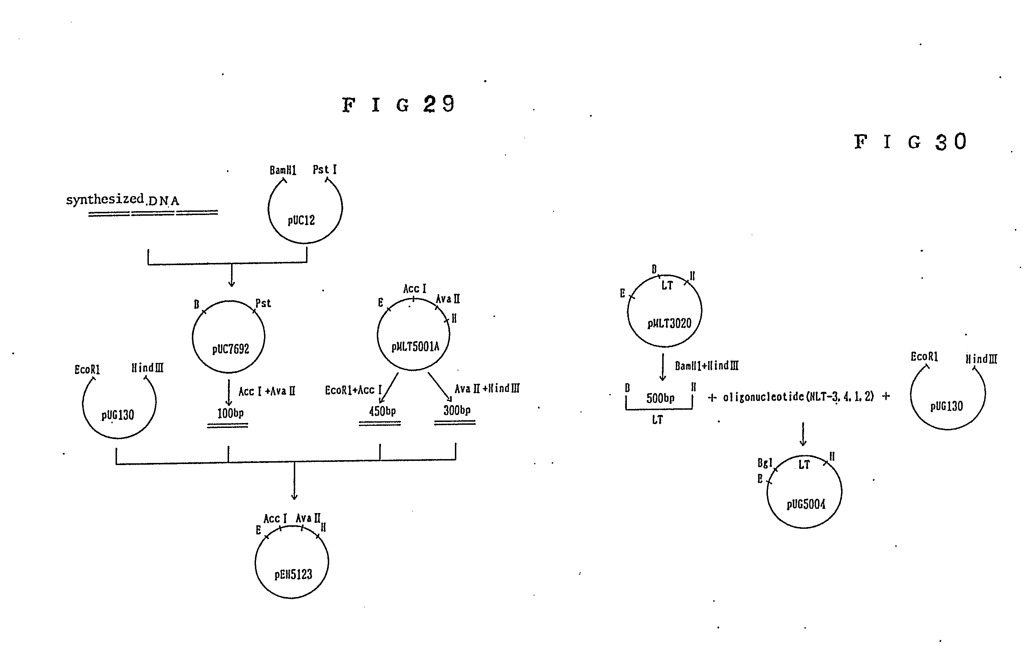

The plasmid pUC7692 was digested with Acc I and Ava II to recover a DNA fragment of about 100 bp from the resulting system by 7% polyacrylamide electrophoresis. Separately, plasmid pMLT5001A was digested with EcoRl and Acc I and with Ava II and Hind III to obtain a DNA fragment of about 450 bp and a DNA fragment of about 300 bp, respectively, while plasmid pUG130 was digested with EcoRl and Hind III to obtain a fragment of about 2.8 kbp. These four fragments were enzymatically joined (Plasmid pEH5123). The flow diagram of this assembly is shown in Fig. 29.

Plasmid pEH5123 was digested with restriction enzymes Acc III and Xho I and treated with bacterial alkaline phosphatase to carry out the dephosphorylation at the 5 terminal.

Subsequently, a fragment of about 3.5 kbp was recovered from the resulting system by agarose gel electrophoresis ( 0.8 %) and enzymatically linked with the following synthetic DNA with 5 terminal phosphorylated ( Plasmid No. pEH6084 ).

5' CCGGAGCTACC 3'

3' TCGATGGAGCT 5'

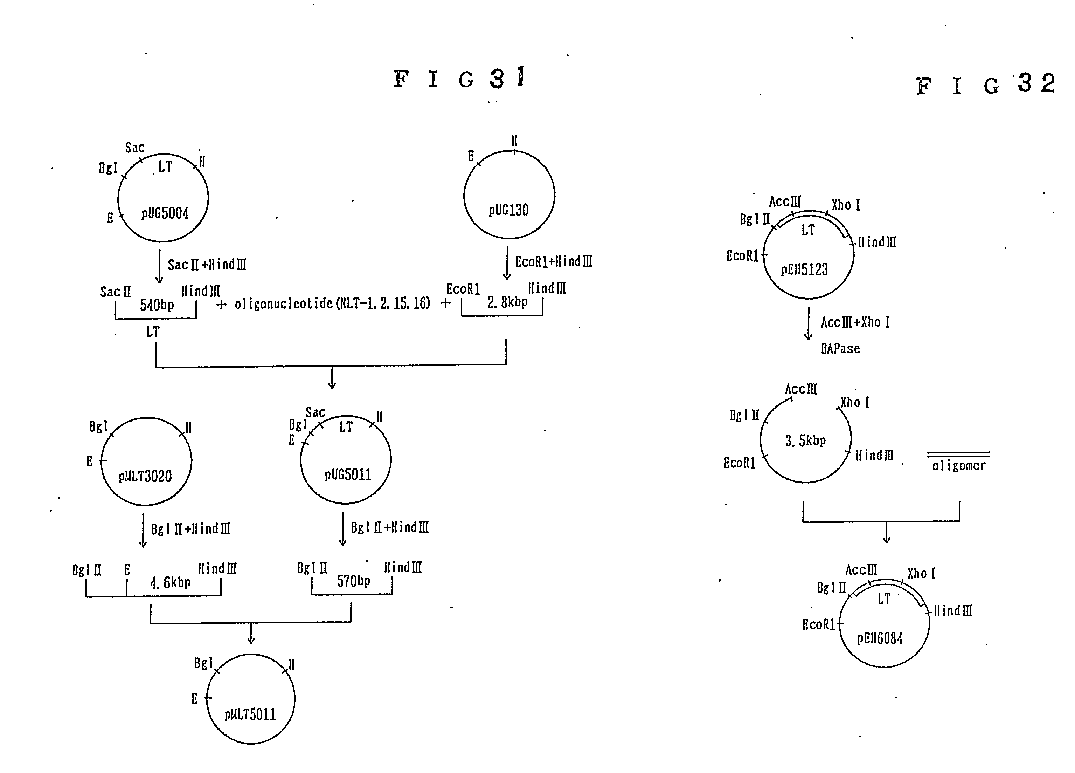

The flow diagram of the assembly is shown in Fig. 32.

### Example 27

A fragment of about 300 bp obtained by cleaving plasmid pEH6084 by restriction enzymes EcoR I and Bg ℓ II, a fragment of about 500 bp obtained by cleaving the plasmid by Sac II and Hind III and the following synthetic DNA with 5 terminal phosphorylated were ensymatically inserted into the site of EcoR I and Hind III in plasmid pUG130 ( Plasmid No.pEH7084 ).

5'     GATCTCATGAGCACCCTCAAACCCGC     3'

3'     AGTACTCGTGGGAGTTTGGG     5'

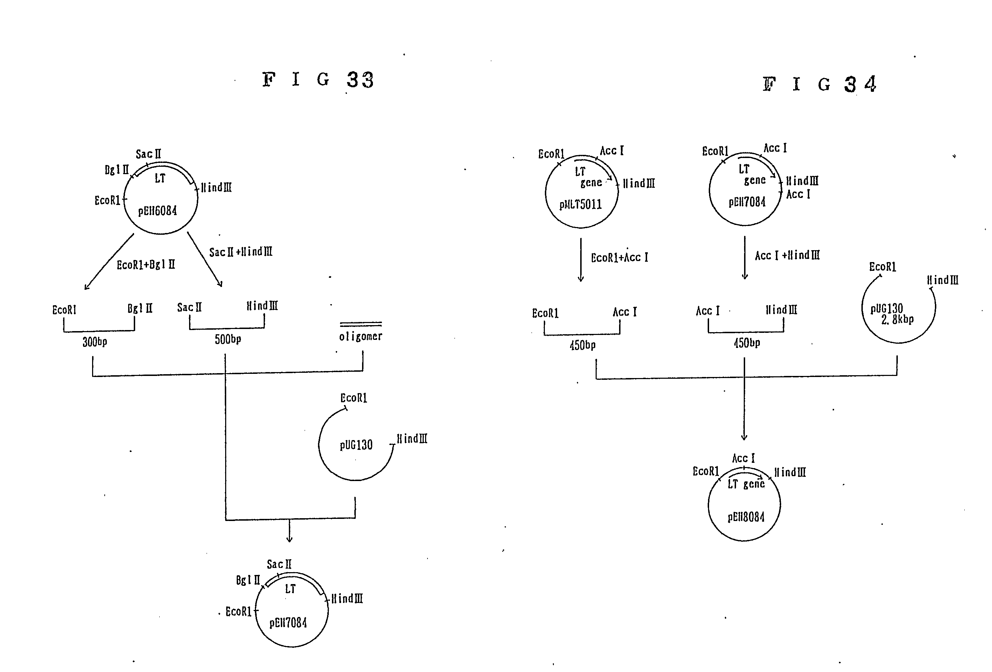

The flow diagram of the assembly is shown in Fig.33.

### Example 28

A DNA fragment of about 500 bp obtained by digesting pMLT3020 by BamHl and Hind III was enzymatically linked with the following synthetic oligonucleotide with 5 terminal phosphorylated and with the vector obtained by digesting pUG130 by EcoRl and Hind III ( Plasmid No pUG5004 ).

The flow diagram of the assembly is shown in Fig.30.

5' AATTCAGATCTCATGCGTCGTCGTAAA 3' NTL-3

3' GTCTAGAGTACGCAGCAGCATTTGGGCGC 5' NTL-4

5' CCCGCGGCTCATCTGATCGGG 3' NTL-1

3' CGAGTAGACTAGCCCCTAG 5' NTL-2

A DNA fragment of about 540 bp obtained by digesting plasmid pUG5004 with Sac II and Hind III, the following synthetic oligonucleotide with 5' terminal phoshorylated and a DNA fragment of about 2.8 kbp obtained by digesting plasmid pUG130 with EcoR I and Hind III were enzymatically linked ( Plasmid No. pUG5011 ).

Subsequently, a DNA fragment of about 570 bp obtained by digesting plasmid pUG5011 with Bg ℓ II and Hind III and a DNA fragment of about 4.6 kbp obtained by digesting plasmid pMLT3020 with Bgℓ II and Hind III were ensymatically linked ( Plasmid No. pMLT5011 ).

The flow diagram of the assembly is shown in Fig. 31.

5' AATTCAGATCTCATGCCCGC 3' NTL-15

3' GTCTAGAGTACGGG 5' NTL-16

Plasmid pEH7084 was digested with restriction enzymes Acc I and Hind III to recover a DNA fragment of about 450 bp from the resulting system by agarose gel electrophoresis ( 1 % ). A DNA fragment of about 450 bp similarly obtained by digesting plasmid pMLT5011 with restirction enzymes EcoR I and Acc I and said fragment were enzymatically inserted into the site of EcoR I and Hind III of plasmid pUG130 ( Plasmid No. pEH8084 ).

The flow diagram of the assembly is shown in Fig. 34.

### Example 29

A DNA fragment of about 580 bp obtained by cleaving plasmid pEH6084 with EcoR I and Pst I, a DNA fragment of about 1700 bp obtained by cleaving pTT5001 with Pst I and Pvu I and a DNA fragment of about 3100 bp obtained by cleaving pBR322 d-rop with EcoR I and Pvu I were enzymatically linked ( Plasmid No. pBRD6084 ).

Subsequently, a DNA fragment of about 1100 bp obtained by cleaving pBRD6084 with EcoR I, Acc I and Pvu I, a DNA fragment of about 450 bp obtained by cleaving pEH8037 with EcoR I and Acc I and a DNA fragment of about 3100 bp obtained by cleaving pBR322 d-rop with EcoR I and Pvu I were enzymatically linked ( Plasmid No. pBRD8050 ).

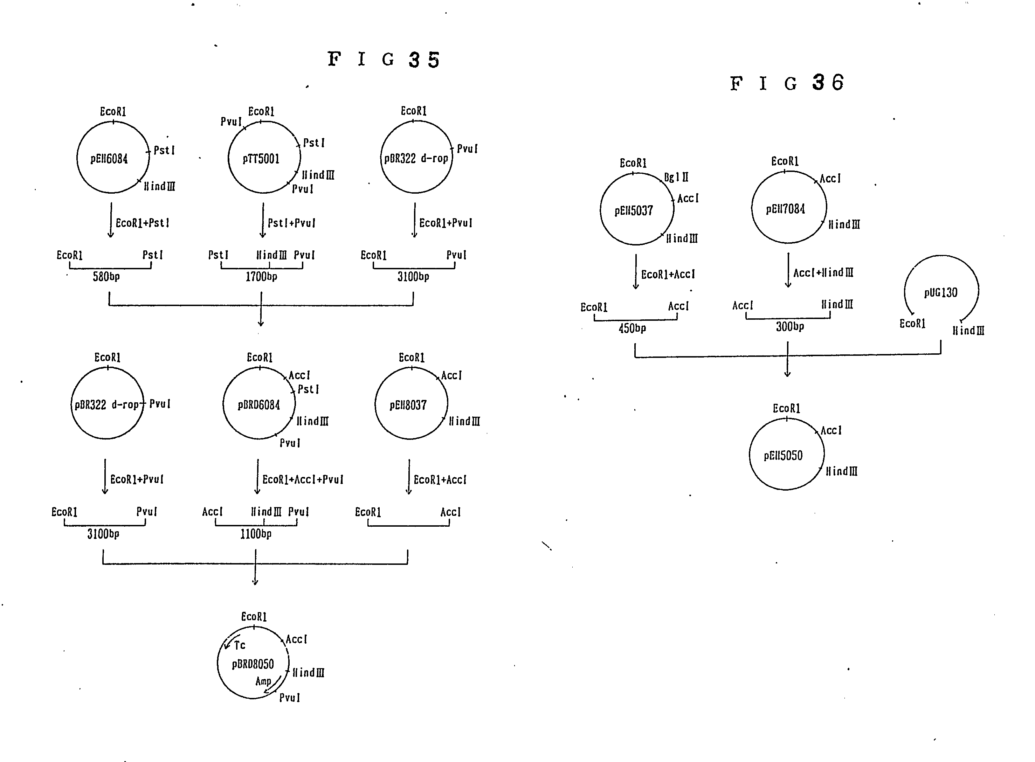

The flow diagram of the assembly is shown in Fig. 35.

### Example 30

Plasmid pEH7084 was cleaved with restriction enzymes Acc I and Hind III to obtain a DNA fragment of about 300 bp. Also. a DNA fragment of about 450 bp obtained by cleaving plasmid pEG5037 with EcoR I and Acc I was inserted enzymatically with said DNA fragment into the site of EcoR I and Hind III of plasmid pUG130 ( Plasmid No. pEH5050 ).

The flow diagram of the assembly is shown in Fig.36.

### Example 31

E. coli W3110 transformed by plasmid pEH6084 was inoculated on 20 t of LB medium containing 100 mg/ℓ of ampicillin and cultured under continuous stirring at 37° C for 20 hours. Then, the cultured medium was centrifuged and the cells were collected followed by washing with PBS, to be suspended in 100 m of PBS. After the disintegration of this suspension of the cells with a sonicator and the centrifuge ( Tomy Seiko : RB20BH, 15,000 rpm, 30 minutes ), 125 m of the curde extract of the cells was obtained. This crude extract exhibited an lymphotoxin activity of 4.4 x 10⁸ units/mℓ. And then, the extract was applied to a column of Sephacryl S-200 ( ⌀ 113 x 900 mm : Pharmacia ) of a gel filtration medium which had been sufficiently equilibrated with 0.2 M phosphate buffer previously. After the fraction which exhibited the lymphotoxin activity was collected, a portion of said fraction was applied on the column of 30 m of Sepharose conjugated with monoclonal mouse anti-lymphotoxin antibodies. The column conjugated with the antibodies was prepared by binding the monoclonal antibodies to Sepharose 4B ( Pharmacia ) ; the monoclonal antibodies were prepared by immunizing a mouse with recombinant lymphotoxin. The column was sufficiently equilibrated with 0.2 M phosphate buffer, followed by the adsorption of the previously purified lymphotoxin derivative and washed with the above buffer. Said lymphotoxin derivative was eluted with 0.2 M phosphate buffer containing 3 M KSCN. Then, the eluate was desalted in Sephadex G-25 ( Pharmacia ) and the purity of the resulting fraction was analyzed by SDS-polyacrylamid gel electrophoresis to be a single band with a molecular weight of approximately 16.000. In addition, the amino acid sequence analysis of the NH₂ terminal showed the following structure : Met Lys Pro Ala Ala His Leu Ile Gly Asp.

### Example 32

E. coli JM83 transformed with plasmid pEH7084 was inoculated on 200 m of LB medium containing 100mg/ℓ of ampicillin and cultured under continuous shaking at 37 °C for 18 hours. Then, the cells collected by centrifuge were washed by PBS (Dulbecco ) and suspended in 2 mℓ of PBS. After this suspension of cells was disintegrated with a sonicator, the crude extract of the cells was obtained by centrifuge. The filtrate obtained after the crude extract was aseptically filtered ( 0.22 µm) was applied to a column of 2 m monoclonal mouse anti-lymphotoxin antibody - Sepharose. The lymphotoxin derivative described above was adsorbed to the column sufficiently equilibrated with 50 mM Tris-HCI ( pH 8.0 ), followed by washing with said buffer. Then, the adsorbed lymphotoxin derivative was eluted with 50 mM Tris-HCI ( pH 8.0 ) containing 3M KSCN. The eluate was immediately dialyzed with PBS ( Dulbecco ).

### Example 33

E. coli JM83 transformed with plasmid pEG8084 was inoculated on 200 m t of LB medium containing 100 mg, ℓ of ampicillin and cultured under continuous shaking at 37 °C for 18 hours. Then. the cells collected by centrifuge were washed by PBS ( Dulbecco ) and suspended in 2 mℓ of PBS. After this suspension of cells was disintegrated with a sonicator, the crude extract of the cells was obtained by centrifuge. Then. the lymphotoxin activity of the crude extract was 6.6 x 10⁷ units/mℓ. The filtrate obtained after the crude extract was aseptically filtered ( 0.22 µm) was applied to a column containing 2 ml of the monoclonal mouse antilymphotoxin antibodies. The lymphotoxin derivative was eluted in a similar procedure as described above and dialyzed immediately with PBS ( Dulbecco ) to obtain 2 m of the dialyzed fraction containing an activity of 2.9 x 10⁷ units/mℓ. The solution has a single band with a molecular weight of about 16,000 determined by SDS-polyacrylamide gel electrophoresis and the sequence of NH₂ terminal was : Pro Ala Ala His Leu lie Gly Asp.

The effect of the present invention will be described by referring to the following Experimental Example.

### Experimental Example 1

The plasmides prepared in the above-described Examples were expressed according to the same procedure as the one described in Example 16 and purified to obtain final object substances. These substances were examined for antitumor activity. The results are shown in Table 2, wherein the plasmids which were used to prepare the final object substances are shown in the column of Plasmid. while the antitumor activity (specific activity based on pMLT5001A) of the substances is shown in the column of Antitumor activity.

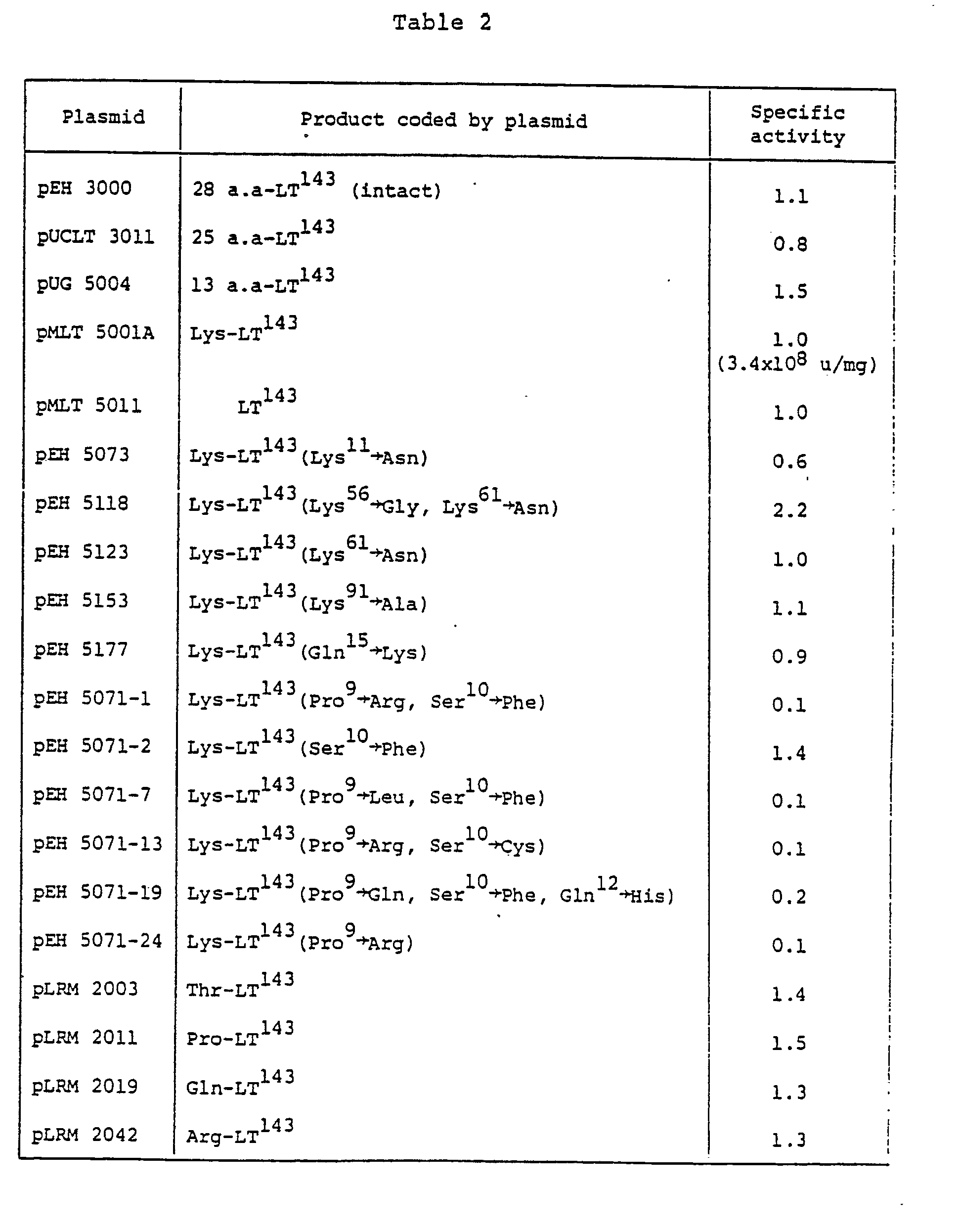

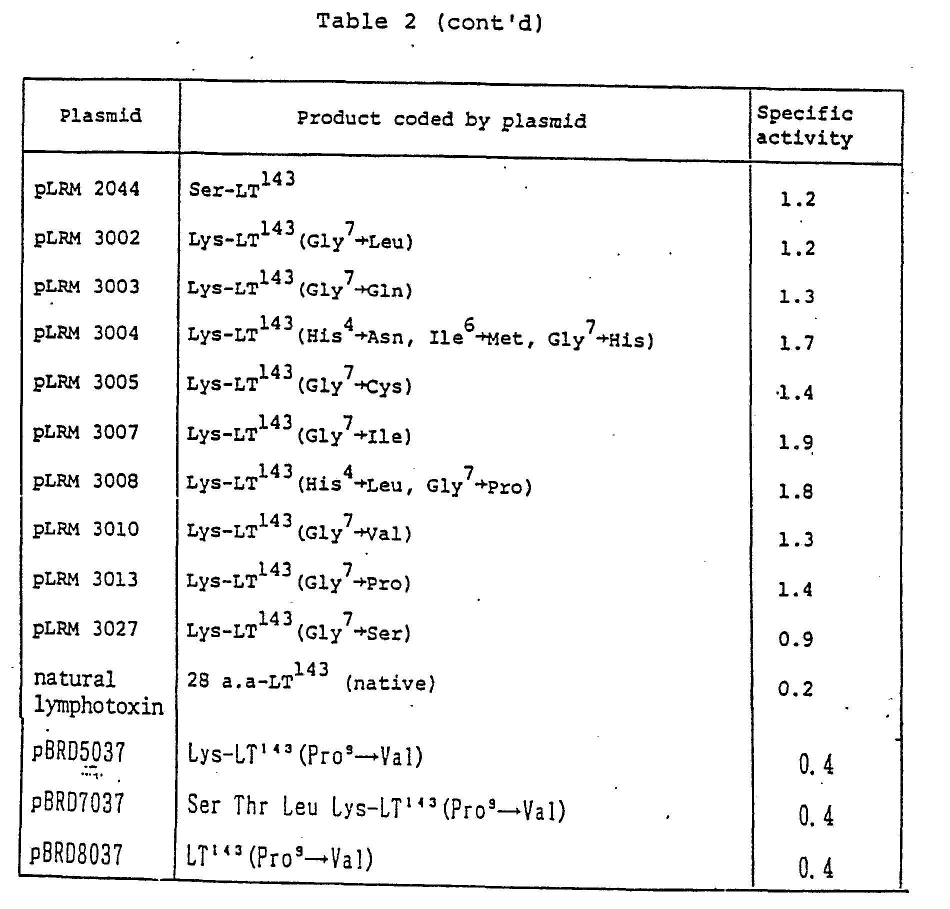

It can be understood from the results shown in Table 2 that the antitumor activity of lymphotoxin resides in the sequence of 143 amino acids according to the present invention.

### Experimental Example 2

### (sample)

Expressive plasmids of pEH5123, pEH5118, pMLT5001A and pBRD7037 were purified to obtain the corresponding substances A. B. C and D, respectively, which were to use as a subject sample. Separately a control sample of natural lymphotoxin was prepared.

### (method)

Human pulmonary cancer cels (PC-10) and human colic adenocarcinoma cells (WiDr) were used as indicator cells. The cells of each cell line were added to a 96 - well microtiter plate at a density of 2 to 5 x 10⁴ well, followed by the addition of each medium containing a subject sample or a control sample and actinomycin D ( 0.1 µg/ml of actinomycin D was added to PC - 10 and 0.5 µg/ml of actinomycin D was added to WiDR) The resulting system was cultured in the presence of humidified C0₂ for 20 hours and the remaining living cells were fixed and stained with crystal violet, followed by the extraction of the dye from the stained cells for measurement of absorbance. The survival rate of the cells containing the sample was calculated, when the number of the living cells before the addition of the sample was regarded as 100 %.

### (results)

Fig. 25 shows test results on anticancer activity (0.1 µg/mℓ Actinomycin D) to human pulmonary cancer cells (PC-10) of pEH5118, pEH5123, pMLT5001 A and natural lymphotoxin. Fig. 25 shows that pEH5118 and pEH5123 are noteworthy owing to their citotoxic activity on cancerous cells, which suggests that the replacement and elimination of the amino acids 56 to 61 might give more useful compounds.

Fig. 26 shows test resutls on anticancer activity (0.5 µg/m Actinomycin D) to human colic adenocarcinoma cells (WiDr) of pBRD7037, pMLT5001 A and natural lymphotoxin. It is noted that pBRD7037 has its citotoxic activity to cancerous cells.

### Experimentanl Example 3

### test on PGE2-breeding-inducing power

### (sample)

Expressive plasmids of pMLT5001A, pBRD7037 and pBRD5037 were purified to obtain the corresponding intended substances C, D and E which were to use as a subject sample. Separately control samples of natural lymphotoxin and recombinant TNF were prepared.

### (method)

Periosteum cells of rats. having been cultivated for 2 to 3 generations after taken, were added to a 96 well microtiter plate at a density of 1 x 10⁴ per one well and cultivated for 4 to 5 hours. The cultivation medium was absorbed and removed out and a new cultivation medium (10% FCS-RPMI-1640) was replaced for that at 50µ well. Then each sample, .diluted to given concentrations in advance, was added thereto in an amount of 50µ ℓ well. Cultivation was conducted at 37° c for 12 hours with 5% of carbon dioxide gas. The supernatant fluid was recovered. An amount of prostaglandin E2 (PGE2) contained in the supernatant fluid was determined with RIA KIT (Dupont NEN research product).

### (results)

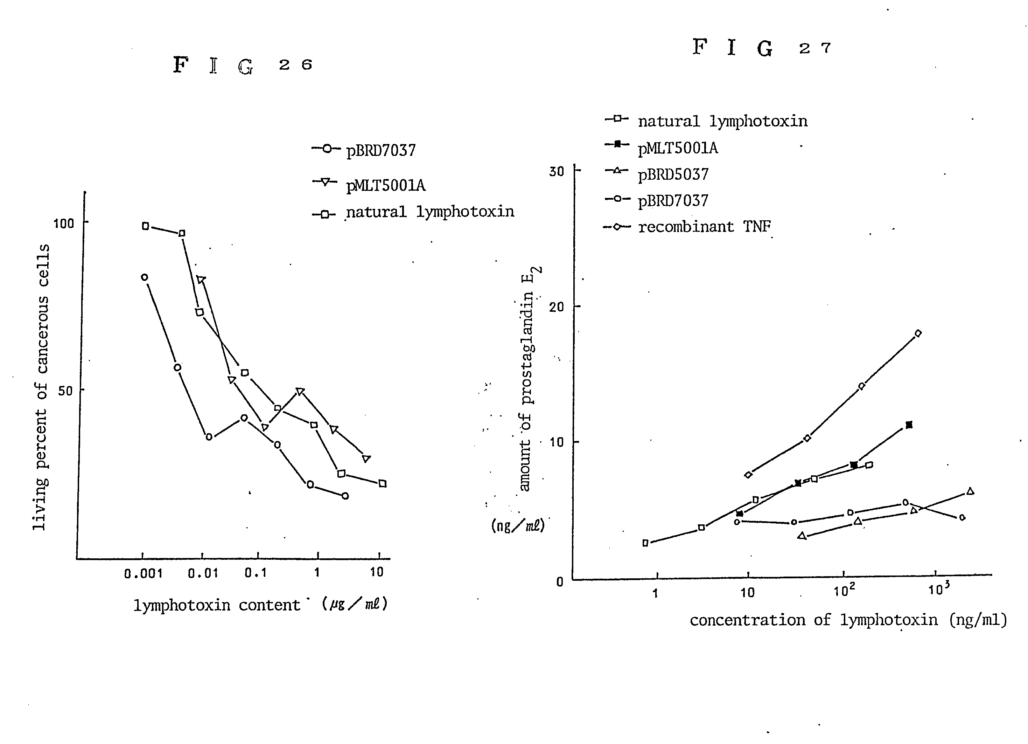

Fig. 27 shows that the recombinant TNF had the strongest prostaglandin E2-breeding-inducing power and natural lymphotoxin and the recombinant lymphotoxin (substance C) were weaker than that. The substances D and E of the invention were more decreased. Accordingly it is understood that the recombinant lymphotoxin of the invention is expected to provide only weak subsidiary ill effects on its clinical practice.

### Experimental Example 4

### < Sample >

The expressive plasmid pEH6084 was purified to obtain the final substance A as a subject sample. Alternatively, as a control sample, a sample of natural lymphotoxin was prepared.

### < Method >

Human pulmonary cancer cells (PC - 10 ), human colic adenocarcinoma cells (WiDr ) and human fibroma cells ( HT 1080) were used as indicator cells. The cells of each cell line was added to a 96 - well microtiter plate at a density of 2 to 5 x 10⁴ well, followed by the addition of each medium containing a subject sample or a control sample and actinomycin D ( 0.1 µg/ml of actinomycin D was added to PC - 10 and 0.5 µg/ml of actinomycin D was added to WiDR and HT 1080. The resulting system was cultured in the presence of humidified C0₂ for 12 to 15 hours and the remaining living cells were fixed and stained with crystal violet, followed by the extraction of the dye from the stained cells for measurement of absorbance. The survival rate of the cells containing the sample was calculated, when the number of the living cells before the addition of the sample was regarded as 100 %.

### < Result >

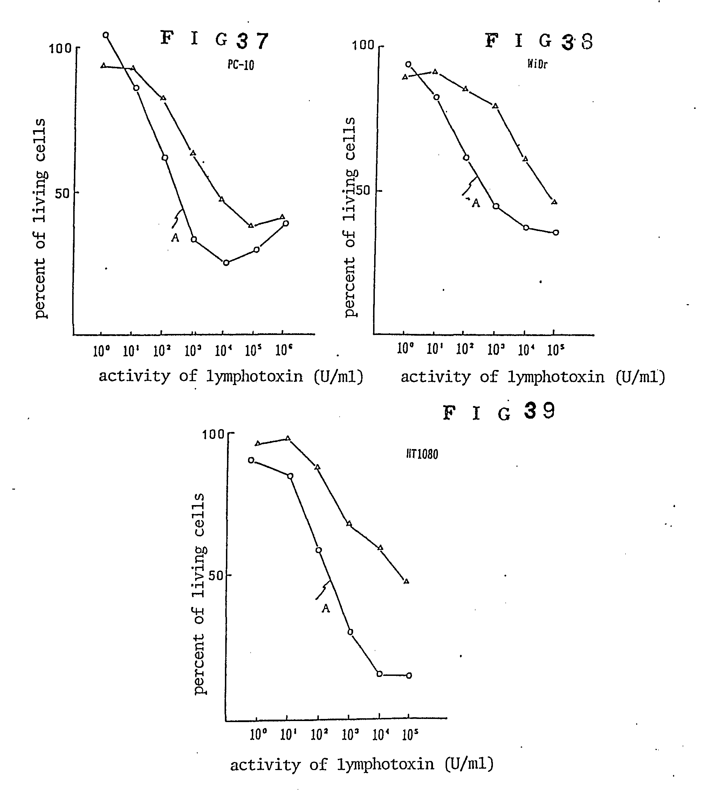

The resutls are shown in Fig. 37 to Fig. 39. Fig. 37 to Fig.39 shows the results obtained using PC - 10, WiDr and HT1080, respectively, as indicator cells ; in the figures, the symbol A and the symbol 0 show the relationship between the lymphotoxin activity and the survival rate in the control sample of natural lymphotoxin and in the sample of the object substance A, respectively.

It can be considered from the results shown in Fig.37 to Fig.39 that the antitumor activity of lymphotoxin resides in the sequence of 137 amino acids according to the present invention.

### Experimental Example 5

### < Sample >

The expressive plasmid of pEH6084, pEH7084 or pEH8084 was purified to obtain the corresponding substance B, C or D as the final object as a subject sample. Alternatively, as a control sample, a sample of natural lymphotoxin was prepared.

### < Method >

Human pulmonary cancer cells ( PC - 10 ), human colic adenocarcinoma cells ( WiDr ) and human fibroma cells ( HT 1080) were used as indicator cells. The cells of each cell line were added to a 96 - well microtiter plate at a density of 2 to 5 x 10⁴ well, followed by the addition of each medium containing a subject sample or a control sample and actinomycin D ( 0.1 mg/ml of actinomycin D was added to PC - 10 and 0.5 µg/ml of actinomycin D was added to WiDR and HT 1080 ). The resulting system was cultured int he presence of humidified CO₂ for 20 hours and the remaining living cells were fixed and stained with crystal violet, followed by the extraction of the dye from the stained cells for measurement of absorbance. The survival rate of the cells containing the sample was calculated, when the number of the living cells before the addition of the sample was regarded as 100 %.

### < Result >

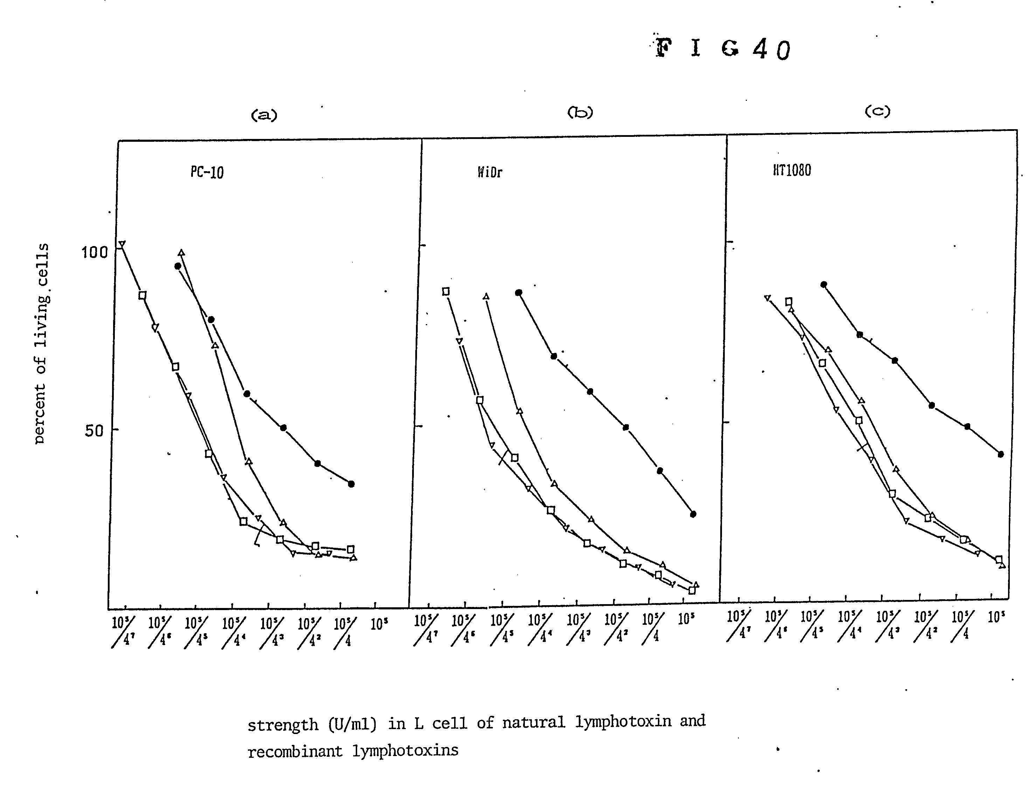

The results are shown in Fig. 40. Fig.40 -(a), (b) and (c) show the results obtained using PC - 10, WiDr and HT1080. respectively, as indicator cells ; in the figures, the symbol •, Δ, a and 0 show the relationship between the lymphotoxin activity and the survival rate in the sample of natural lymphotoxin, the object substance B, C and D, respectively.

It can be considered from the results shown in Fig.40 -(a), (b) and (c) that the antitumor activity of lymphotoxin resides in the sequence of 137 amino acids according to the present invention.

### Experimental Example 6

The activity that the sample of B, C or D described in the Experimental Example 5 had against mouse L-P3 cells was determined by enzyme immunoassay ( 0.5 - 1 x 10⁸ units/ mg protein ) and the bioactivity of each sample was calculated by using the activity of natural lymphotoxin as the standard.

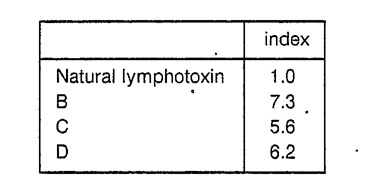

It can be considered by the above described results that the activity of the lymphotoxin derivative according to the present invention has 5 to 8 fold that of natural lymphotoxin.

## Claims

1. A recombinant lymphotoxin derivative containing, in its molecule, an amino acid sequence represented by the following primary structural formula: 
10
Pro Ala Ala A1 Leu A2 A3 Asp A4 A5 A6 Gln
20
Asn Ser Leu A7 A8 A9 A10 Asn Thr A11 A12
30
Ala Phe Leu Gln Asp Gly Phe Ser Leu Ser
40
A13 Asn Ser Leu Leu Val Pro Thr Ser Gly
50
Ile Tyr Phe Val Tyr Ser Gln Val Val Phe
60
Ser A14 A15 A16 A17 A18 A19 A20 A21 Thr
70
Ser Ser Pro Leu Tyr Leu Ala His Glu Val
80
Gln Leu Phe Ser Ser Gln Tyr Pro Phe His
90
Val Pro Leu Leu Ser Ser Gln A22 Met Val
100
Tyr Pro Gly Leu Gln Glu Pro Trp Leu His
110
Ser Met Tyr His Gly Ala Ala Phe Gln Leu
120
Thr A23 A24 A25 A26 Leu Ser Thr His Thr
130
Asp Gly Ile Pro His Leu Val Leu A27 A28
140
A29 A30 Val A31 Phe Gly Ala Phe Ala Leu
 wherein A_{1~31} may be each any amino acid.

2. A recombinant lymphotoxin derivative as set forth in claim 1, wherein A₁ is His, Asn or Leu; A₂ is lie, Met or Gly; A3 is Leu, Gin, His, Cys, lie, Pro, Val, Ser or Gly; A₄ is Pro, Arg, Leu, Gln or Val; As is Ser, Phe or Cys; A₆ is Lys, Asn, Gin, Glu or Asp; A₇ is Leu, Ala or Gin; A₈ is Trp, Phe or Leu; As is Arg or Leu; A₁₀ is Ala, Arg or Glu; A₁₁ is Asp, Asn, Ala or Arg; A₁₂ is Arg, Asn or Gly; A, ₃ is Asn, ARg, Asp, Ala or Ser; A₁₄ is Gly, Arg, Lys, Pro, Ala, Ser or Thr; A₁₅ is Lys, Gly, Ala, Thr or Asn; A₁ is Ala, Arg, Lys, Glu, Gin, Ser or Gly; A₁₇ is Try, Lys, Arg, Cys or Gly; A₁₈ is Ser, Arg, Lys. Phe, Thr or Ala; A₁ is Pro, Lys. Ser, Thr or Gln; A₂₀ is Lys, Asn, Gly or Thr; A₂₁ is Ala, Lys, Ser or Glu; A₂₂ is Lys or Asn; A₂₃ is Gin, Lys, Arg or Gly; A24 is Gly, Asp or Ser: A₂₅ is Asp, Glu or Gln; A₂₆ is Gln, Arg, Pro or Ser; A₂₇ is Ser, Thr, Glu, Phe or Trp; A₂₈ is Pro, Thr, Trp or Tyr; A₂₉ is Ser, Thr or Tyr, A₃₀ is Thr, Val or Phe and A₃₁ is Phe or Tyr.

3. A recombinant lymphotoxin derivative as set forth in claim 1 or 2, wherein A₁ is His; A₂ is Ile; A3 is Gly; A₄ is Pro or Val; As is Ser; A₆ is Lys; A₇ is Leu; A₈ is Trp; A₉ is Arg; A₁ is Ala; A₁₁ is Asp; A₁ is Arg; A₁₃ is Asn; A₁ is Gly; A₁₅ is Lys; A₁₆ is Ala; A₁₇ is Tyr; A₁ is Ser; A₁ is Pro; A₂₀ is Lys; A₂₁ is Ala; A₂₂ is Lys; A₂₃ is Gin; A₂₄ is Gly; A₂₅ is Asp; A₂₆ is Gin; A₂₇ is Ser; A₂₈ is Pro; A₂₉ is Ser; A₃₀ is Thr and A₃₁ is'Phe.

4. A recombinant lymphotoxin derivative as set forth in any of claims 1 to 3, wherein the polypeptide joined to the amino terminal of said amino acid sequence comprises 1 to 28 amino acid residues.

5. A cDNA coding the recombinant lymphotoxin derivative as defined in Claim 1.

6. An expressive plasmid which contains, as an exogenote, a cDNA coding the recombinant lymphotoxin derivative as defined in Claim 1.

7. An expressive plasmid as claimed in Claim 6, in which the plasmid is selected from the following group:

pEH3000, pUCLT3011, pMLT5011, pMLT5001A,
pUG5004, pEH5073, pEH5118, pEH5123,
pEH5153, pEH5177, pEH5071-1, pEH5071-2,
pEH5071-7, pEH5071-13, pEH5071-19,
pEH5071-24, pEH5096, pLRM3002, pLRM3003,
pLRM3004, pLRM3005, pLRM3007, pLRM3008,
pLRM3010, pLRM3013, pLRM3027, pLRM2003,
pLRM2011, pLRM2019, pLRM2042, pLRM2044,
pBRD5037, pBRD7037, pBRD7071, pBRD8037,
pBRD8071

8. A host transformed with an expressive plasmid which contains, an exogenote, a cDNA coding the recombinant lymphotoxin derivative as defined in Claim 1.

9. A host as claimed in Claim 8, in which the plasmid is selected from the following group;

pEH3000, pUCLT3011, pMLT5011, pMLT5001A,
pUG5004, pEH5073, pEH5118, pEH5123,
pEH5153, pEH5177, pEH5071-1, pEH5071-2,
pEH5071-7, pEH5071-13, pEH5071-19,
pEH5071-24, pEH5096, pLRM3002, pLRM3003,
pLRM3004, pLRM3005, pLRM3007, pLRM3008,
pLRM3010, pLRM3013, pLRM3027, pLRM2003,
pLRM2011, pLRM2019, pLRM2042, pLRM2044,
pBRD5037, pBRD7037, pBRD7071, pBRD8037,
pBRD8071

10. A pharmaceutical composition which comprises a pharmacologically effective amount of the recombinant lymphotoxin as defined in Claim 1 and a pharmacologically acceptable carrier.

11. A recombinant lymphotoxin derivative containing, in its molecule, an amino acid sequence having the following primary structural formula: 
Pro Ala Ala A1 Leu A2 A3 Asp A4 A5 A6 Gln
Asn Ser Leu A7 A8 A9 A10 Asn Thr A11 A12
Ala Phe Leu Gln Asp Gly Phe Ser Leu Ser
A13 Asn Ser Leu Leu Val Pro Thr Ser Gly
Ile Tyr Phe Val Tyr Ser Gln Val Val Phe
Ser A14 A21 Thr Ser Ser Pro Leu Tyr Leu
Ala His Glu Val Gln Leu Phe Ser Ser Gln
Tyr Pro Phe His Val Pro Leu Leu Ser Ser
Gln A22 Met Val Tyr Pro Gly Leu Gln Glu
Pro Trp Leu His Ser Met Tyr His Gly Ala
Ala Phe Gln Leu Thr A23 A24 A25 A26 Leu
Ser Thr His Thr Asp Gly Ile Pro His Leu
Val Leu A27 A28 A29 A30 Val A31 Phe Gly
Ala Phe Ala Leu
 in which A₁ to A₁₄ and A_{2'} to A₃₁ each are an amino acid.

12. A recombinant lymphotoxin derivative as claimed in Claim 11, in which A1 is His, Asn or Leu; A₂ is Ile, Met or Gly; A3 is Leu, Gin, His, Cys, Ile, Pro, Val, Ser or Gly; A₄ is Pro, Arg, Leu, Gln or Val; As is Ser, Phe or Cys; A₆ is Lys, Asn, Gin, Glu or Asp; A₇ is Leu, Ala or Gin; A₈ is Trp, Phe or Leu; A₉ is Arg or Leu; A₁₀ is Ala, Arg or Glu; A₁ is Asp, Asn, Ala or Arg; A₁₂ is Arg, Asn or Gly; A₁₃ is Asn, ARg, Asp, Ala or Ser; A₁₄ is Gly, Arg, Lys, Pro, Ala, Ser or Thr; A₂₁ is Ala, Lys, Ser or Glu; A₂₂ is Lys or Asn; A₂₃ is Gin, Lys, Arg or Gly; A₂₄ is Gly, Asp or Ser; A₂₅ is Asp, Glu or Gin; A₂₆ is Gin, Arg, Pro or Ser; A₂₇ is Ser, Thr, Glu, Phe or Trp; A₂₈ is Pro, Thr, Trp or Tyr; A₂₉ is Ser, Thr or Tyr, A₃₀ is Thr, Val or Phe and A₃₁ is Phe or Tyr.

13. A recombinant lymphotoxin derivative as claimed in Claim 11, in which A1 is His; A2 is Ile; A₃ is Gly; A₄ is Pro or Val; A₅ is Ser; A₆ is Lys; A₇ is Leu; A₈ is Trp; A₉ is Arg; A₁₀ is Ala; A₁ is Asp; A₁₂ is Arg; A₁ is Asn; A, ₁₄ is Gly; A₂₁ is Ala; A₂₂ is Lys; A₂₃ is Gin; A₂₄ is Gly; A₂₅ is Asp; A₂₆ is Gln; A₂₇ is Ser; A₂₈ is Pro; A₂₉ is Ser; A₃₀ is Thr and A₃₁ is Phe.

14. A recombinant lymphotoxin derivative as claimed in Claim 11, in which the polypeptide joined to the amino terminal of the amino acid sequence comprises 1 to 28 amino acid residues.

15. A cDNA coding the recombinant lymphotoxin derivative as claimed in Claim 11.

16. An expressive plasmid which contains, as an exogenote, a cDNA coding the recombinant lymphotoxin derivative as defined in Claim 11.

17. A host transformed with an expressive plasmid which contains, an exogenote, a cDNA coding the recombinant lymphotoxin derivative as defined in Claim 11.

18. A pharmaceutical composition which comprises a pharmacologically effective amount of the recombinant lymphotoxin as defined in Claim 11 and a pharmacologically acceptable carrier.
